# EUROPEAN PATENT SPECIFICATION

(11) **EP 1 009 813 B1**
(45) Date of publication and mention of the grant of the patent: **20.06.2007**
(21) Application number: 98940027.0
(22) Date of filing: 20.08.1998
(51) Int. Cl.: C12N 9/10, C12Q 1/68, A01K 67/027

(54) **THE PHYSIOLOGICAL CLOCK-RELATED GENES GRO-1, GOP-1, GOP-2, GOP-3, AND HAP-1**
DIE AUF DER PHYSIOLOGISCHEN UHR BEZOGENEN GENE GRO-1, GOP-2, GOP-2, GOP-3, UND HAP-1
GENES GRO-1, GOP-1, GOP-3 ET HAP-1 ASSOCIE A LA MONTRE PHYSIOLOGIQUE

(30) Priority: 25.08.1997 CA 2210251
(43) Date of publication of application: 21.06.2000
(73) Proprietor: McGill University, Montréal, Québec H3A 1B1 (CA)
(72) Inventor: HEKIMI, Siegfried, Montréal, Québec H4P 1E5 (CA); LAKOWSKI, Bernard, Genzentrum, Ludwig-Maximillians, D-81377 Munich (DE); BARNES, Thomas, Boston, MA 02118 (US); LEMIEUX, Jason, Toronto, Ontario M6G 2M2 (CA)
(74) Representative: Evans, Claire
(86) International application number: PCT/CA1998/000803
(87) International publication number: WO 1999/010482

(56) References cited:
- WILSON R ET AL: "2.2 MB OF CONTIGUOUS NUCLEOTIDE SEQUENCE FROM CHROMOSOME III OF C ELEGANS" NATURE, vol. 368, no. 6466, 3 March 1994, pages 32-38, XP002029739 -& DATABASE EMBL - CEZC395 Entry CEZC395, Acc.No. U13642, 30 November 1994 WILSON, R. ET EL.: "Caenorhabditis elegans cosmid ZC395" XP002089006 -& DATABASE EMBL - EMINV Entry CEC34E10, Acc.No. U10402, 30 June 1994 WILSON, R. ET EL.: "Caenorhabditis elegans cosmid C34E10" XP002089545
- ADAMS M D ET AL: "INITIAL ASSESSMENT OF HUMAN GENE DIVERSITY AND EXPRESSION PATTERNS BASED UPON 83 MILLION NUCLEOTIDES OF CDNA SEQUENCE" NATURE, vol. 377, 28 September 1995, pages 3-17, XP002042918 -& DATABASE EMBL - EMEST14 Entry HSZZ37212, Acc.No. AA332152, 18 April 1997 ADAMS, M.D. ET AL.: "EST36068 Embryo, 8 week I Homo sapiens cDNA 5' end similar to similar to tRNA isopentenyltransferase." XP002089546 -& DATABASE EMBL - EMEST14 Entry HSZZ61218, Acc.No. AA356092, 18 April 1997 ADAMS, M.D. ET AL.: "EST64588 Jurkat T-cells VI Homo sapiens cDNA 5' end similar to similar to tRNA isopentenyltransferase." XP002089547
- LAKOWSKI, B. ET AL.: "Determination of life-span in Caenorhabditis elegans by four clock genes." SCIENCE, vol. 272, 17 May 1996, pages 1010-3, XP002089004 cited in the application
- EWBANK, J.J. ET AL.: "Structural and functional conservation of the Caenorhabditis elegans timing gene clk-1." SCIENCE, vol. 275, 14 February 1997, pages 980-3, XP002089005 cited in the application
- SPIETH, J. ET AL.: "Operon in C. elegans: polycistronic mRNA precursors are processed by trans-splicing of SL2 to downstream coding regions." CELL, vol. 73, 1993, pages 521-32, XP002089544 cited in the application

## Description

### BACKGROUND OF THE INVENTION

### (a) Field of the Invention

The invention relates to the identification of *gro-1* gene and four other genes located within the same operon and to show that the *gro-1* gene is involved in the control of a central physiological clock.

### (b) Description of Prior Art

The *gro*-1 gene was originally defined by a spontaneous mutation isolated from of a *Caenorhabditis elegans* strain that had recently been established from a wild isolate (J. Hodgkin and T. Doniach, Genetics 146: 149-164 (1997)). We have shown that the activity of the *gro*-*1* gene controls how fast the worms live and how soon they die. The time taken to progress through embryonic and post-embryonic development, as well as the life span of *gro-1* mutants is increased (Lakowski and Hekimi, Science 272:1010-1013, (1996)). Furthermore, these defects are maternally rescuable: when homozygous mutants *(gro-1*/*gro-1)* derive from a heterozygous mother *(gro-1*/*+),* these animals appear to be phenotypically wild-type. The defects are seen only when homozygous mutants derive from a homozygous mother (Lakowski and Hekimi, Science 272:1010-1013, (1996)). In general, the properties of the *gro-1* gene are similar to those of three other genes, *clk-1, clk-2* and *clk-3* (Wong et al., Genetics 139: 1247-1259 (1995); Hekimi et al., Genetics, 141: 1351-1367 (1995); Lakowski and Hekimi, Science 272:1010-1013, (1996)), and this combination of phenotypes has been called the Clk ("clock") phenotype. All four of these genes interact to determine developmental rate and longevity in the nematode. Detailed examination of the *clk*-*1* mutant phenotype has led to the suggestion that there exists a central physiological clock which coordinates all or many aspects of cellular physiology, from cell division and growth to aging. All four genes have a similar phenotype and thus appear to impinge on this physiological clock.

It would be highly desirable to be provided with the molecular identity of the *gro-1* gene.

### SUMMARY OF THE INVENTION

One aim of the present invention is to provide the molecular identity of the *gro-1* gene and four other genes located within the same operon.

In accordance with a first aspect of the present invention, there is provided an isolated polynucleotide that encodes a polypeptide having the amino acid sequence encoded by the nucleotide sequence of SEQ ID NO:3 or a complement of said polynucleotide.

Preferably the isolated polynucleotide has a nucleotide sequence of SEQ ID NO:3 or a complement thereof.

In accordance with a second aspect of the present invention, there is provided a fragment of the polypeptide encoded by the isolated polypeptide of the first aspect of the invention, wherein said fragment can rescue at least one phenotype in a *gro-1 (e2400) C. elegans* mutant.

The polynucleotide preferably further comprises one or more nucleotide sequences encoding a heterologous polypeptide. The heterologous polypeptide is preferably green fluorescent protein (GFP).

The *gro-1* gene is located with an operon which has the nucleotide sequence set forth in SEQ ID NO: 1 and which also codes for four other genes, referred to as *gop-1, gop-2, gop-3,* and *hap-1* genes.

In accordance with a third aspect of the present invention, there is provided a *gro-1* protein encoded by the polynucleotide of the first or second aspect of the present invention.

In accordance with a fourth aspect of the present invention, there is provided a *gop-3* gene which codes for a *gop-3* protein having the amino acid sequence set forth in SEQ ID NO:6, wherein said *gop-3* gene is located in the *gro-1* operon and said *gop-3* gene is transcriptionally co-expressed with *gro-1* gene present in said operon. Also provided is a *gop-3* protein encoded by same, having the amino acid sequence set forth in SEQ ID NO: 6.

In accordance with a fifth aspect of the present invention, there is provided a *hap-1* gene which codes for a *hap-1* protein having the amino acid sequence set forth in SEQ ID NO:7, wherein said *hap-1* gene is located in the *gro-1* operon and said *hap-1* gene is transcriptionally co-expressed with *gro-1* gene present in said operon. Also provided is a *hap-1* protein encoded by same and having the amino acid sequence set forth in SEQ ID NO: 7.

In accordance with a sixth aspect of the present invention, there is provided a mutant *gro-1* protein comprising the amino acid sequence of HAEYINHSKY GVTLVLKNSF HGSIWTHQKW IHSMGINCSS KDAMM.

In accordance with a seventh aspect of the present invention, there is provided *a gro-1* protein having the amino acid sequence of SEQ ID NO:2.

In accordance with a eighth aspect of the present invention, there is provided a method for the diagnosis and/or prognosis of cancer in a sample from a patient, which comprises analyzing DNA of said patient in said sample to determine if the human *gro-1* gene sequence is altered in comparison to the human *gro-1* nucleotide sequence set forth in SEQ ID NO:3, wherein alteration of the human *gro-1* gene is indicative of cancer.

In accordance with a ninth aspect of the present invention, there is provided a mouse model of aging and cancer, which comprises a gene knock-out of the murine *gro-1* gene that is homologous to the polypeptide of the first aspect of the present invention.

In accordance with a tenth aspect of the present invention, there is provided the use of a phosphate nucleotide for the manufacture of a medicament for interfering with enzymatic activity of *gro-1* of the third or seventh aspect of the present invention.

Also provided is a vector comprising the polynucleotide of same.

The vector preferably comprises the gene of the fourth or fifth aspects of the present invention.

The vector preferably is an expression vector.

Also provided is a host cell which comprises the above vector.

### BRIEF DESCRIPTION OF THE DRAWINGS

Fig. 1A illustrates the genetic mapping of *gro-1;*
Fig. 1B illustrates the physical map of the *gro-1* region;
Fig. 2A illustrates cosmid clones able to rescue the *gro-1 (e2400)* mutant phenotype;
Fig. 2B illustrates the genes predicted by Genefinder, the relevant restriction sites and the fragments used to subclone the region;
Figs. 3A-3C illustrate the genomic sequence and translation of the *C*. *elegans gro-1* gene (SEQ. ID. NO:2) ;
Fig. 3D illustrates the predicted mutant protein;
Fig. 4A illustrates the five genes of the *gro-1* operon (SEQ. ID. NO:1);
Fig. 4B illustrates the transplicing pattern of the five genes of the *gro-1* operon;
Fig. 5A-5B illustrate the alignment of *gro-1* with the published sequences of the *E*. *coli* (P16384) and yeast (P07884) enzymes;
Fig. 6 illustrates the biosynthetic step catalyzed by DMAPP transferase (MiaAp in *E*. *coli,* Mod5p in *S. cerevisiae,* and GRO-1 in *C*. *elegans);*
Fig. 7 illustrates the alignment of the predicted HAP-1 amino acid sequence with homologues from other species;
Fig. 8 illustrates the full mRNA sequence of human homologue of *gro-1* referred to as hgro-1 (SEQ. ID. NO:3);
Fig. 9A-9B illustrate a comparison of the conceptual amino acid sequences for GRO-1 and hgro-1p;
Fig. 10 illustrates a conceptual translation of a partial sequence of the Drosophila homologue of *gro-1* (AA816785);
Fig. 11A-11B illustrate the structure of pMQ8;
Fig. 12 illustrates construction of pMQ18;
Figs. 13A-13E illustrate the genomic sequence and translation of the *gop-1* gene (SEQ. ID. NO:4);
Fig. 14A-14B illustrate the genomic sequence and translation of the *gop-2* gene (SEQ. ID. NO:5) ;
Figs. 15A-15D illustrate the genomic sequence and translation of the *gop-3* gene (SEQ. ID. N0:6); and
Fig. 16A-16B illustrate the genomic sequence and translation of the *hap-1* gene (SEQ. ID. NO: 7)

### DETAILED DESCRIPTION OF THE INVENTION

### The gro-1 phenotype

In addition to the previously documented phenotypes, we recently found that *gro-1* mutants were temperature-sensitive for fertility. At 25°C the progeny of these mutants is reduced so much that a viable strain cannot be propagated. In contrast, *gro-1* strains can easily be propagated at 15 and 20°C.

We also discovered that the *gro-1(e2400)* mutation increases the incidence of spontaneous mutations. As *gro-1(e2400)* was originally identified in a non-standard background (Hodgkin and Doniach, Genetics 146: 149-164 (1997)), we first backcrossed the mutations 8 times against N2, the standard wild type strain. We then undertook to examine the *gro-1* strain and N2 for the occurrence of spontaneous mutants which could be identified visually. We focused on the two class of mutants which are detected the most easily by simple visual inspection, uncoordinated mutants (Unc) and dumpy mutants (Dpy). We examined 8200 wild type worms and found no spontaneous visible mutant. By contrast, we found 6 spontaneous mutants among 12500 *gro-1* mutants examined. All mutants produced entirely mutant progeny indicating that they were homozygous.

### Sequences of all primers used

| **Name** | **Orientation** | **Sequence (5'-3')** | **SEQ ID NO:** |
|---|---|---|---|
| SHP91 | forward | CGAACACTTTATATTTCTCG | SEQ. ID. NO:8 |
| SHP92 | reverse | GATAGTTCCCTTCGTTCGGG | SEQ. ID. NO:9 |
| SHP93 | forward | TTTCTGGATTTTAACCTTCC | SEQ. ID. NO:10 |
| SHP94 | forward | TTTCCGAGAAGTCACGTTGG | SEQ. ID. NO:11 |
| SHP95 | reverse | TACAGGAATTTTTGAACGGG | SEQ. ID. NO:12 |
| SHP96 | forward | CTTCAGATGACGTGGATTCC | SEQ. ID. NO:13 |
| SHP97 | forward | GGAATCCGAAAAAGTGAACT | SEQ. ID. NO:14 |
| SHP98 | forward | AAGAGATACACTCAATGGGG | SEQ. ID. NO:15 |
| SHP99 | reverse | ATCGATACCACCGTCTCTGG | SEQ. ID. NO:16 |
| SHP109 | reverse | TTGAATCTACACTAATCACC | SEQ. ID. N0:17 |
| SHP100 | reverse | CCAATTATCTTTTCCAGTCA | SEQ. ID. N0:18 |
| SHP110 | forward | ACATTATAAAGTTACTGTCC | SEQ. ID. N0:19 |
| SHP118 | forward | TTTTAGTTAMGCATTGACC | SEQ. ID. NO:20 |
| SHP119 | reverse | ACATCTTTATCCATTTCTCC | SEQ. ID. NO:21 |
| SHP120 | forward | TGCAAAGGCTCTGGAACTCC | SEQ. ID. NO:22 |
| SHP129 | reverse | AAAAACCACTTGATATAAGG | SEQ. ID. NO:23 |
| SHP130 | reverse | CATCCAAAAGCAGTATCACC | SEQ. ID. NO:24 |
| SHP134 | forward | TTAATTGGATGCAAGCACCCC | SEQ. ID. NO:25 |
| SHP135 | reverse | ATTACTATACGAACATTTCC | SEQ. ID. NO:26 |
| SHP138 | forward | TTGTAAAGGCGTTAGTTTGG | SEQ. ID. NO:27 |
| SHP139 | forward | CAGGAGTATTTGGTGATGCG | SEQ. ID. NO:28 |
| SHP140 | forward | CGACGGGGAGAAGGTGACGG | SEQ. ID. NO:29 |
| SHP141 | reverse | AAAACTTCTACCAACAATGG | SEQ. ID. NO:30 |
| SHP142 | reverse | CGTAATCTCTCTCGATTAGC | SEQ. ID. NO:31 |
| SHP143 | reverse | CCGTGGGATGGCTACTTGCC | SEQ. ID. NO:32 |
| SHP144 | reverse | TGGATTTGTGGCACGAGCGG | SEQ. ID. NO:33 |
| SHP145 | reverse | TTGATTGCCTCTCCTCGTCC | SEQ. ID. N0:34 |
| SHP146 | reverse | ATCAACATCTGATTGATTCC | SEQ. ID. NO:35 |
| SHP151 | forward | CAGCGAGCGCATGCAACTATATATTG AGCAGG | SEQ. ID. NO:36 |
| SHP159 | forward | AATAAATATTTAAATATTCAGATATACC CTGAACTCTACAG | SEQ. ID. NO:37 |
| SHP160 | reverse | AAACTGTAGAGTTCAGGGTATATCTG AATATTTAAATATTTATTC | SEQ. ID. N0:38 |
| SHP161 | forward | GTACGTGGAGCTCTGCAACTATATATT GAGCAGG | SEQ. ID. NO:39 |
| SHP162 | reverse | ATGACACTGCAGGATAGTTCCCTTCG TTCGGG | SEQ. ID. NO:40 |
| SHP163 | forward | GTGTTGCATCAGTTCATTCC | SEQ. ID. NO:41 |
| SHP164 | forward | GCTGTGCTAGAAGTCAGAGG | SEQ. ID. NO:42 |
| SHP165 | reverse | GTTCTCCTTGGAATTCATCC | SEQ. ID. NO:43 |
| SHP170 | reverse | AGTATATCTAGATGTGCGAGTCTCTG CCAATT | SEQ. ID. NO:44 |
| SHP171 | reverse | AGTAATTGTACATTTAGTGG | SEQ. ID. NO:45 |
| SHP172 | forward | ATTAACCTTACTTACTTACC | SEQ. ID. NO:46 |
| SHP173 | forward | CTAAACTAAGTAATATAACC | SEQ. ID. NO:47 |
| SHP174 | reverse | GTTGATTCTTTGAGCACTGG | SEQ. ID. NO:48 |
| SHP175 | forward | AATTCGACCAATTACATTGG | SEQ. ID. NO:49 |
| SHP176 | reverse | AACATAGTTGTTGAGGAAGG | SEQ. ID. NO:50 |
| SHP177 | forward | AATTAATGGAGATTCTACGG | SEQ. ID. NO:51 |
| SHP178 | forward | TCAGCATCTAGAAATGCAGG | SEQ. ID. NO:52 |
| SHP179 | reverse | CGAATGTCAACATTCACTGG | SEQ. ID. NO:53 |
| SHP180 | forward | CTTAACCTGATGTGTACTCG | SEQ. ID. NO:54 |
| SHP181 | forward | ATGAAGCTTTAGAGGATGCC | SEQ. ID. NO:55 |
| SHP182 | forward | CGACGAATTTCTGGAGTCGG | SEQ. ID. NO:56 |
| SHP183 | reverse | ACTGCATTATCCATTAATCC | SEQ. ID. NO:57 |
| SHP184 | reverse | CACCCAAATAACATCTATCC | SEQ. ID. NO:58 |
| SHP185 | forward | TTTAACCTCATCTTCGCTGG | SEQ. ID. NO:59 |
| SHP190 | forward | ATGTTCCGCAAGCTTGGTTC | SEQ. ID. NO:60 |
| SL1 | forward | TTTAATTACCCAAGTTTGAG | SEQ. ID. N0:61 |
| SL2 | forward | TTTTAACCCAGTTACTCAAG | SEQ. ID. N0:62 |

### Positional cloning of gro-1

*gro-1* lies on linkage group III, very close to the gene *clk-1.* To genetically order *gro-1* with respect to *clk-1* on the genetic map, 54 recombinants in the *dpy-17* to *lon-1* interval were selected from among the self progeny of a strain which was *unc-79 (e1030) + + c1k-1 (e2519) lon-1 (e678) +*/*+ dpy-17 (e164)* gro-*1 (e2400) + sma-4 (e729).* Three of these showed neither the Gro-1 nor the Clk-1 phenotypes, but carried *unc-79* and sma-4, indicating that these recombination events had occurred between *gro-1* and *clk-1.* From the disposition of the markers, this showed that the gene order was *dpy-17 gro-1 clk-1 lon-1,* and the frequency of events indicated that the *gro-1* to *clk-1* distance was 0.03 map units. In this region of the genome, this corresponds to a physical map distance of -20 kb.

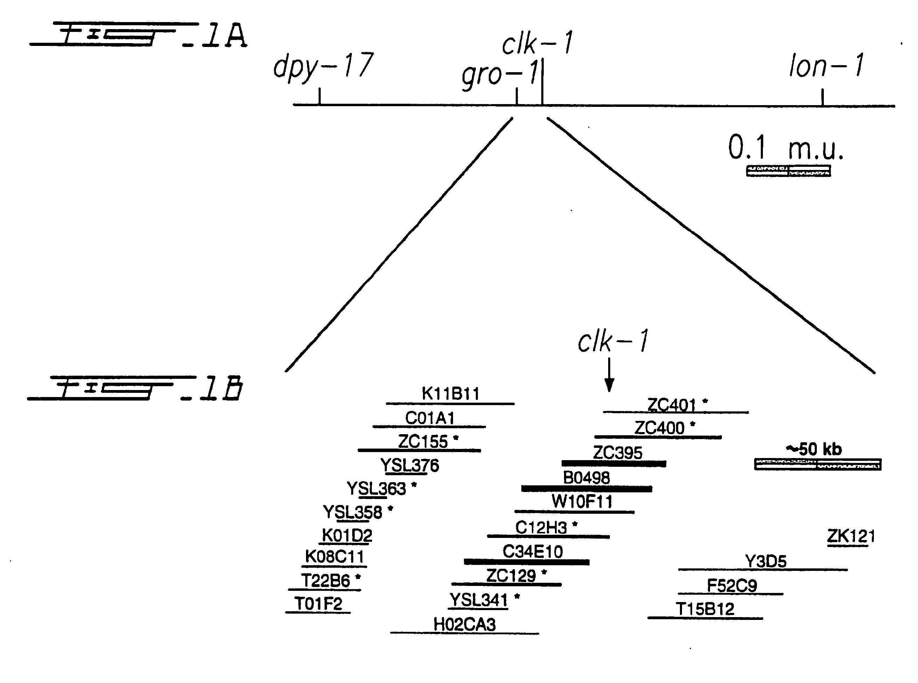

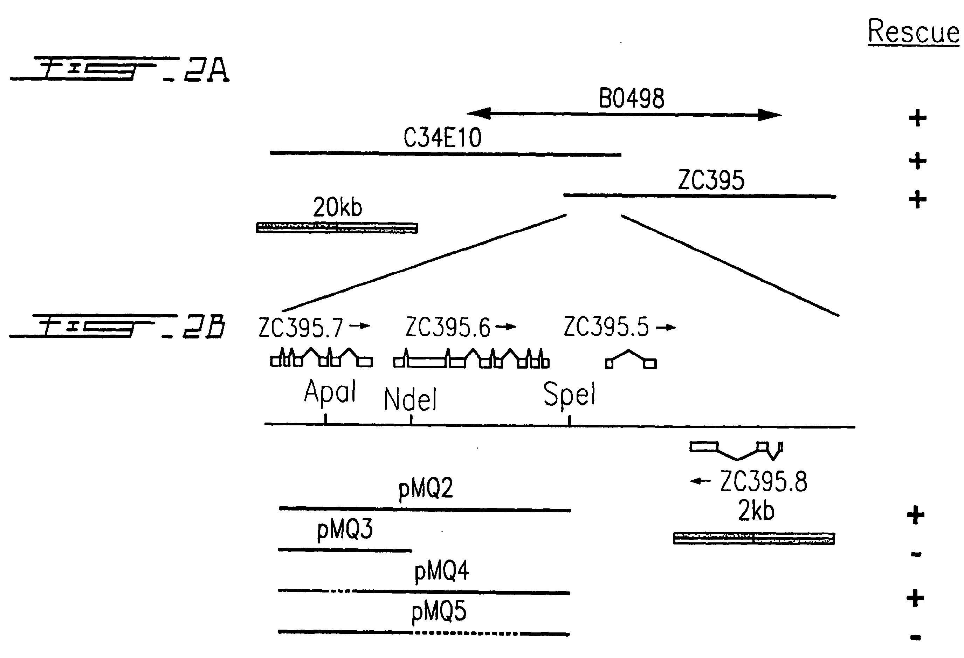

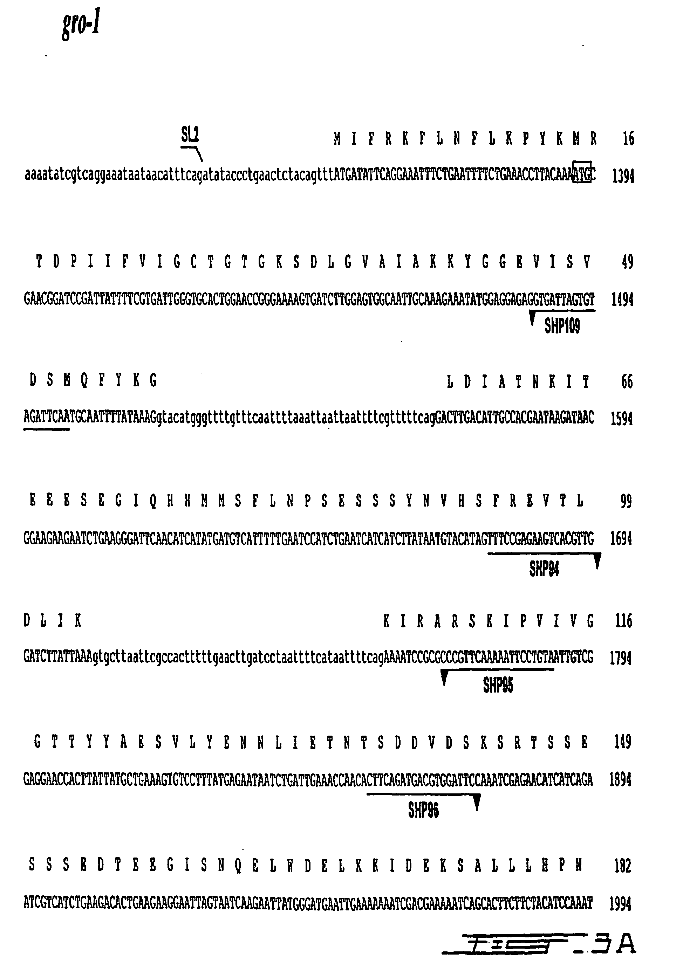

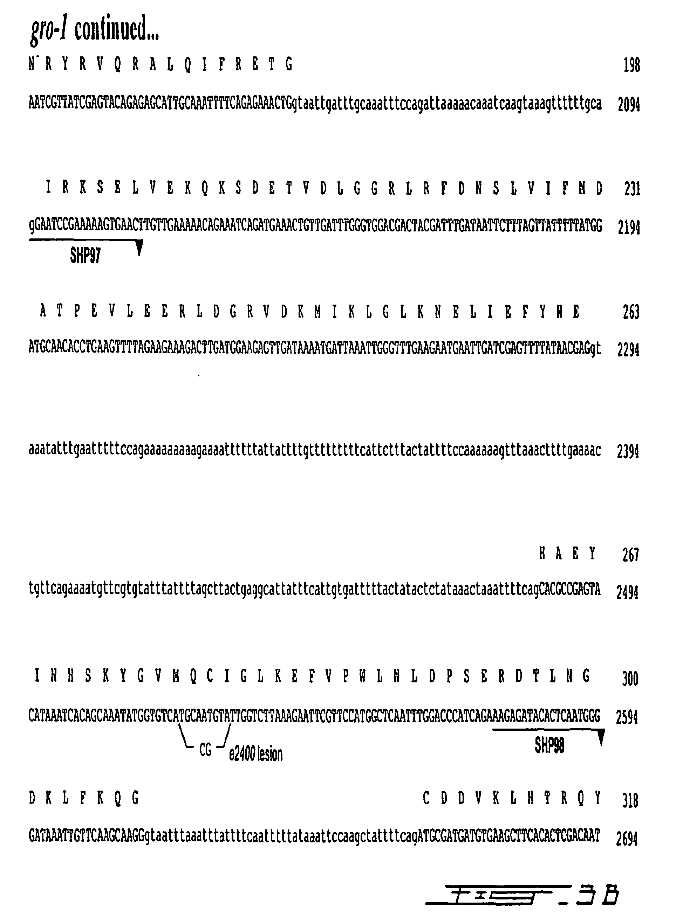

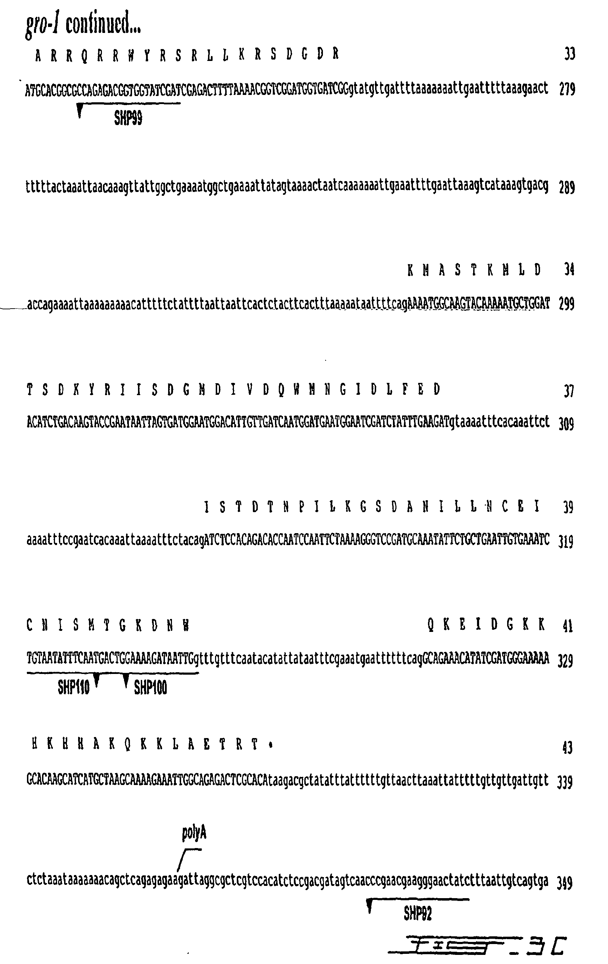

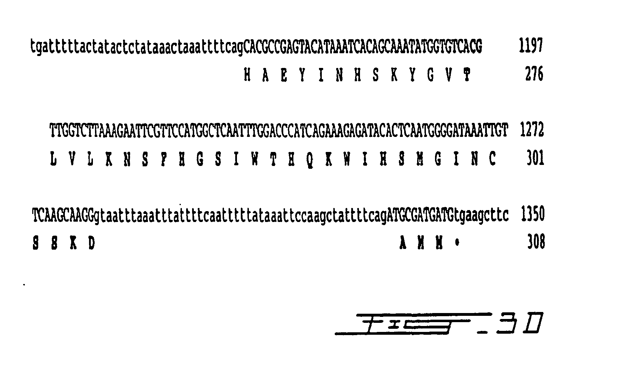

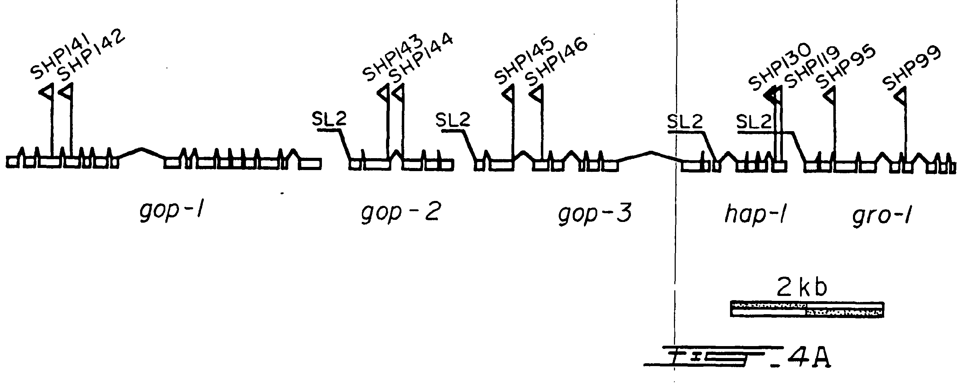

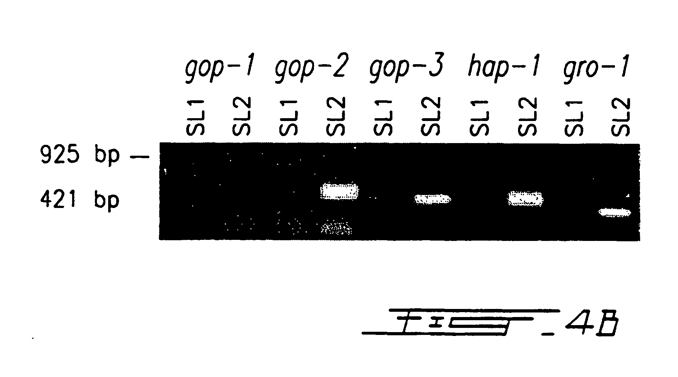

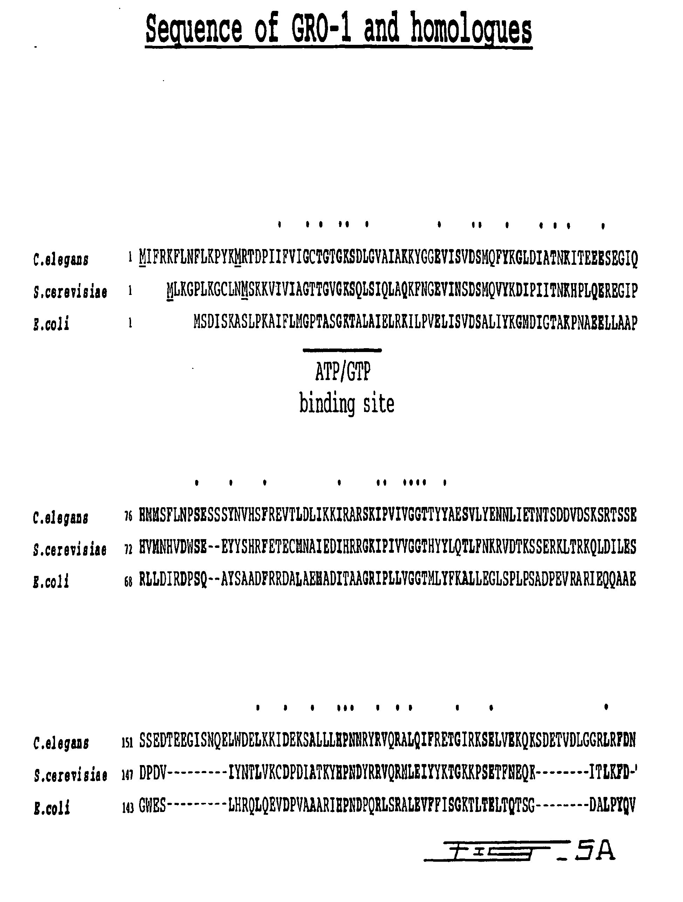

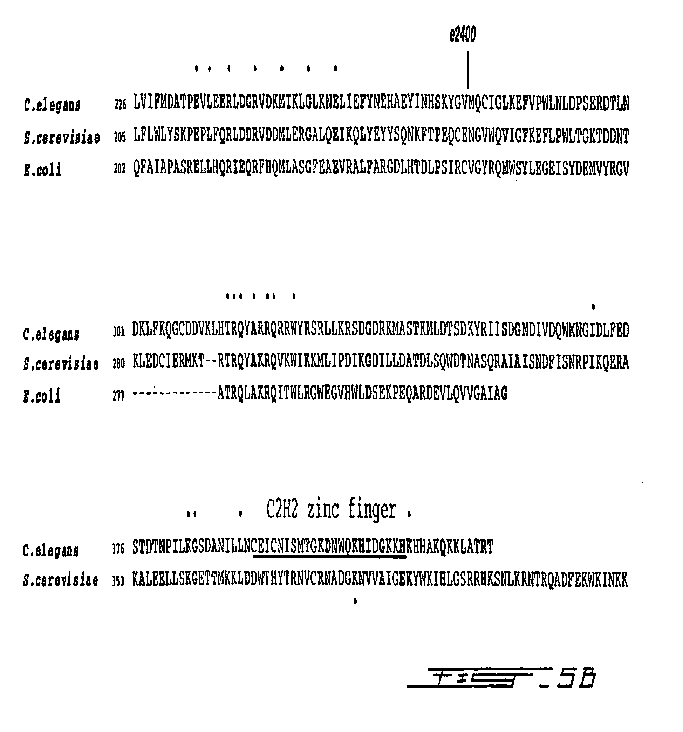

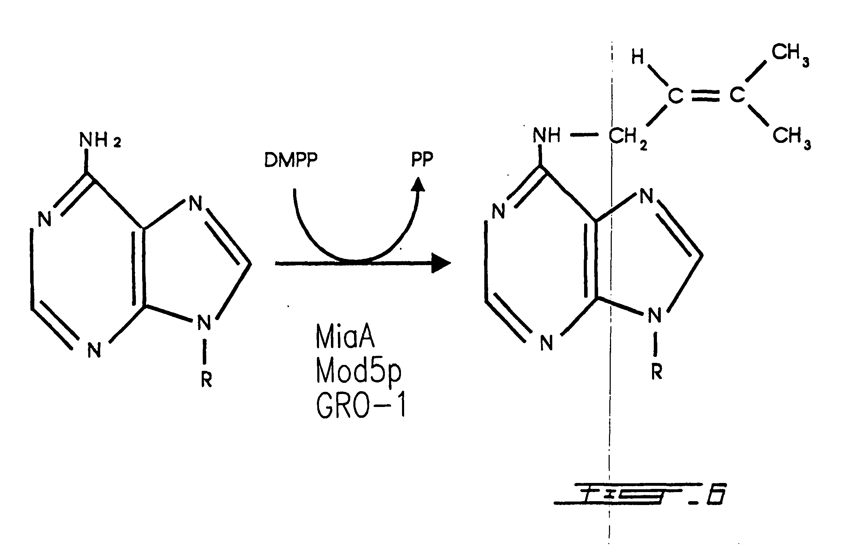

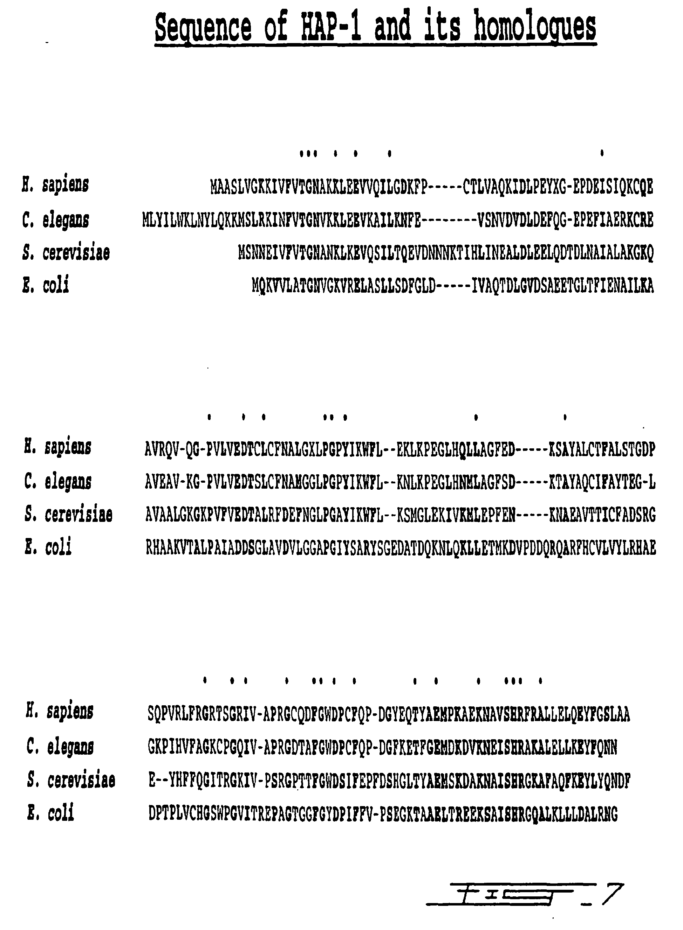

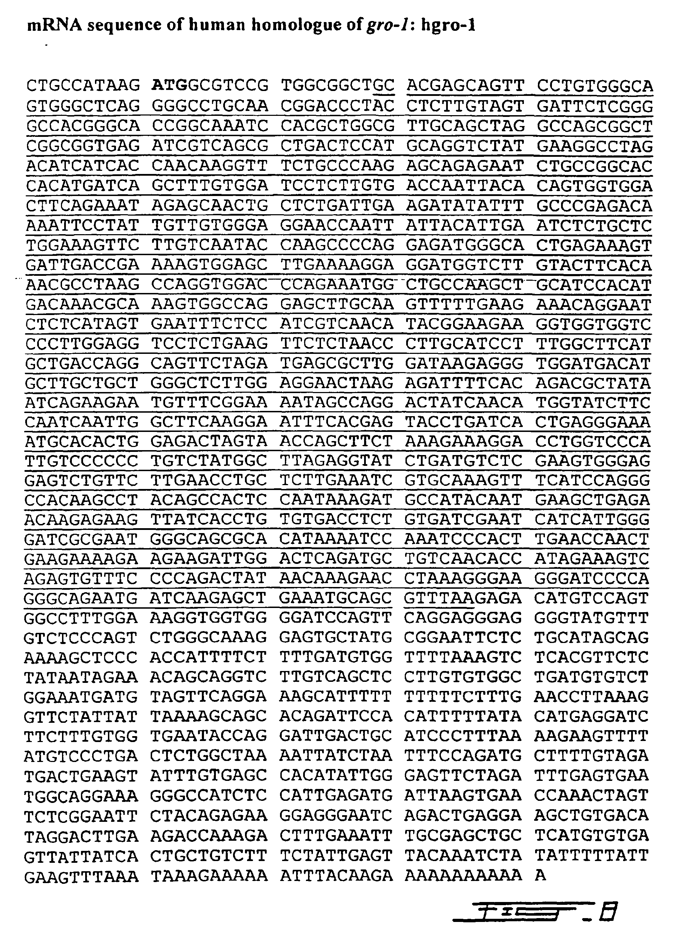

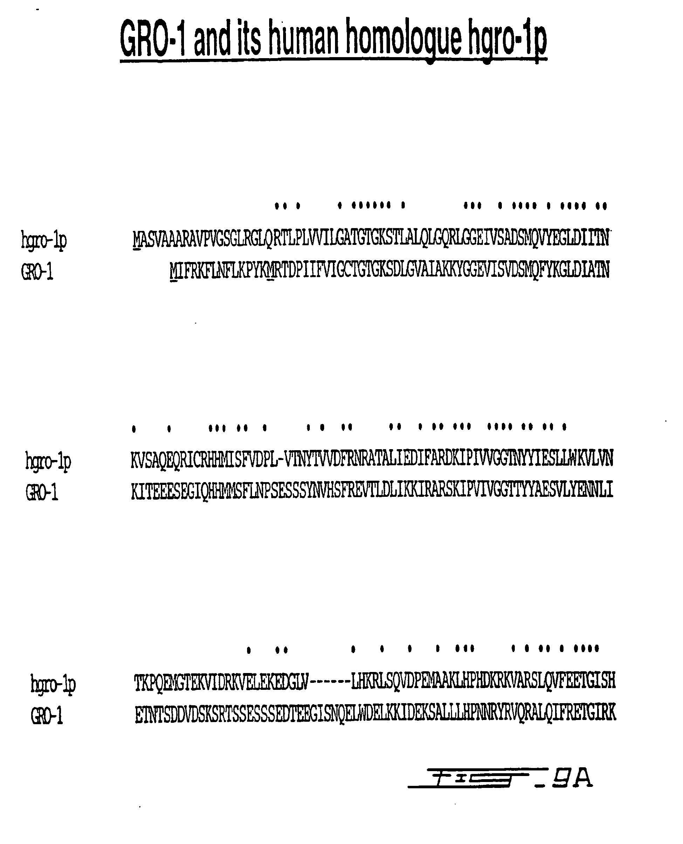

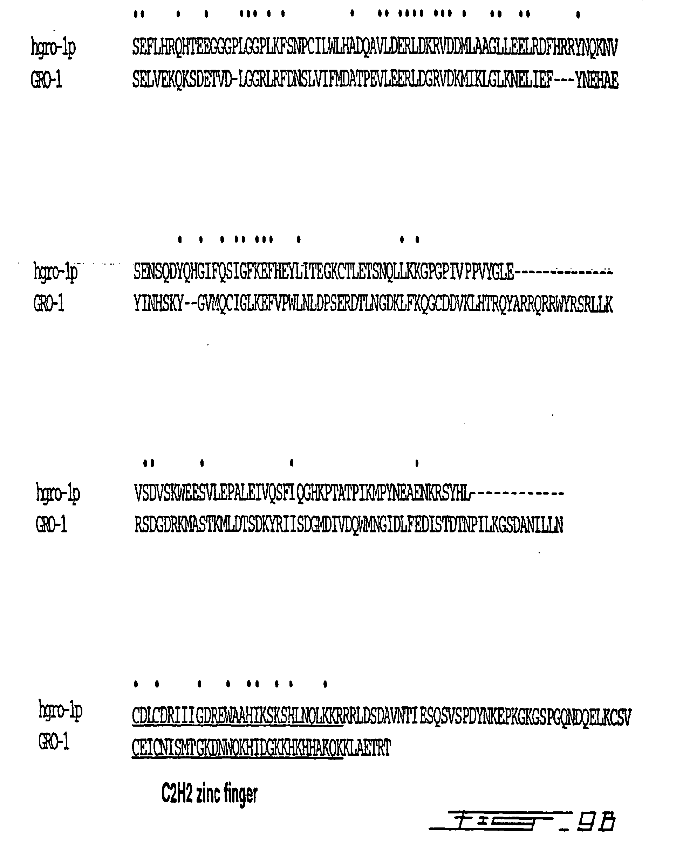

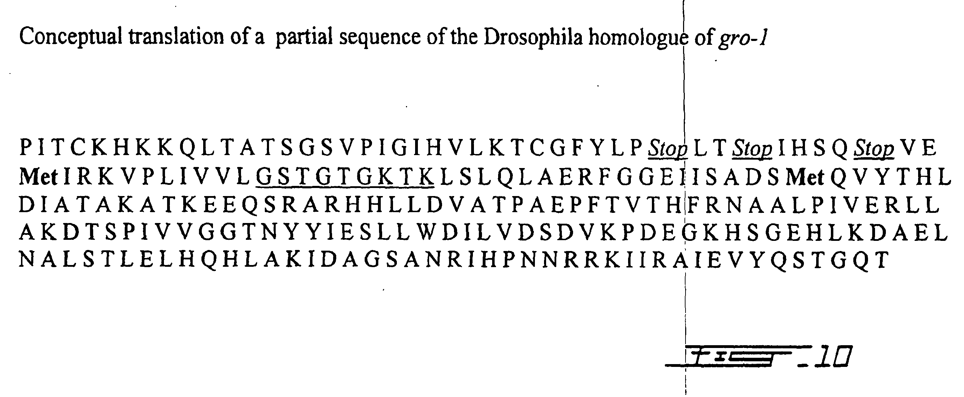

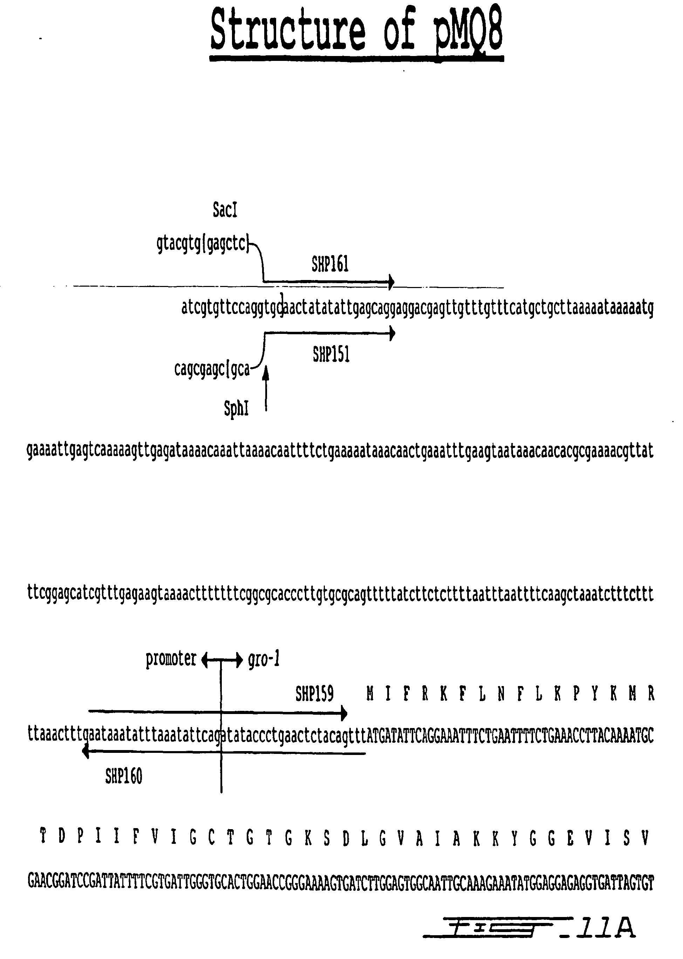

Several cosmids containing wild-type DNA spanning this region of the genome were tested by microinjection into *gro-1* mutants for their ability to complement the *gro-1(e2400)* mutation (Fig. 1). *gro-1* was mapped between *dpy*-*17* and *lon-1* on the third chromosome, 0.03 m.u. to the left of *clk-1* (Fig. 1A).

Based on the above genetic mapping, *gro-1* was estimated to be approximately 20 kb to the left of *clk-1*. Eight cosmids (represented by medium bold lines) were selected as candidates for transformation rescue (Fig. 1B). Those which were capable of rescuing the *gro-1(e2400)* mutant phenotype are represented as heavy bold lines (Fig. 1B).

Of these, only B0498, C34E10 and ZC395 were able to rescue the mutant phenotype. Transgenic animals were fully rescued for developmental speed. In addition, the transgenic DNA was able to recapitulate the maternal rescue seen with the wild-type gene, that is, mutants not carrying the transgenic DNA but derived from transgenic mothers display a wild type phenotype. The 7 kb region common to the three rescuing cosmids had been completely sequenced, and this sequence was publicly available.

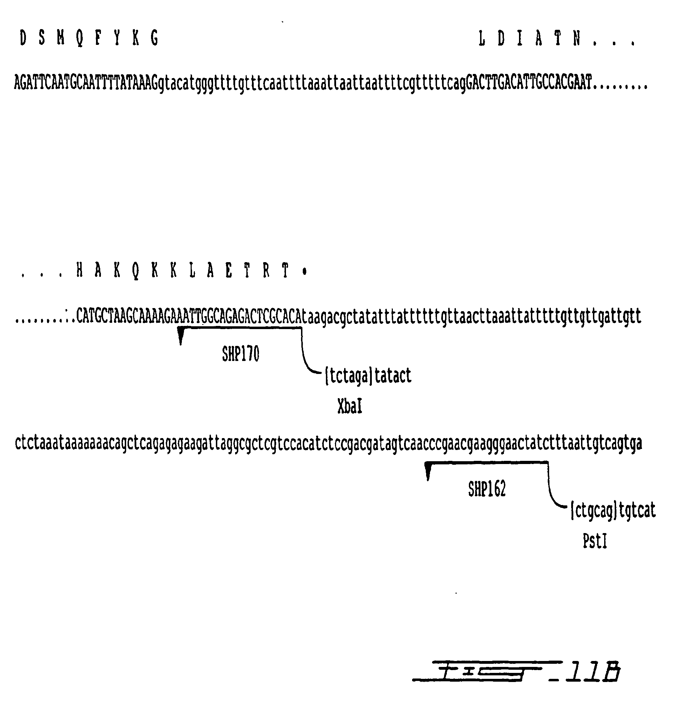

We generated subclones of ZC395 and assayed them for rescue (Fig. 2). The common 6.5 kb region is blown up in part B. B0498 has not been sequenced and therefore its ends can not be positioned and are therefore represented by arrows.

One subclone pMQ2, spanned 3.9 kb and was also able to completely rescue the growth rate defect and recapitulate the maternal effect. The sequences in pMQ2 potentially encodes two genes. However, a second subclone, pMQ3, which contained only the first of the potential genes (named ZC395.7 in Fig. 2A), was unable to rescue.

Furthermore, frameshifts which would disrupt each of the two genes' coding sequences were constructed in pMQ2 and tested for rescue. Disruption of the first gene (in pMQ4) did not eliminate rescuing ability, but disruption of the second gene (in pMQ5) did. This indicates that the *gro-1* rescuing activity is provided by the second predicted gene.

pMQ2 was generated by deleting a 29.9 kb *Spe*I fragment from ZC395, leaving the left-most 3.9 kb region containing the predicted genes ZC395.7 and ZC395.6 (Fig. 2B). pMQ3 was created in the same fashion, by deleting a 31.4 kb NdeI fragment from ZC395, leaving only ZC395.7 intact. In pMQ4, a frameshift was induced in ZC395.7 by degrading the 4 bp overhang of the *Apa*I site. A frameshift was also induced in pMQ5 by filling in the 2 bp overhang of the NdeI site found in the second exon of ZC395.6. These frameshifts presumably abolish any function of ZC395.7 and ZC395.6 respectively. The dotted lines represent the extent of frameshift that resulted from these alterations.

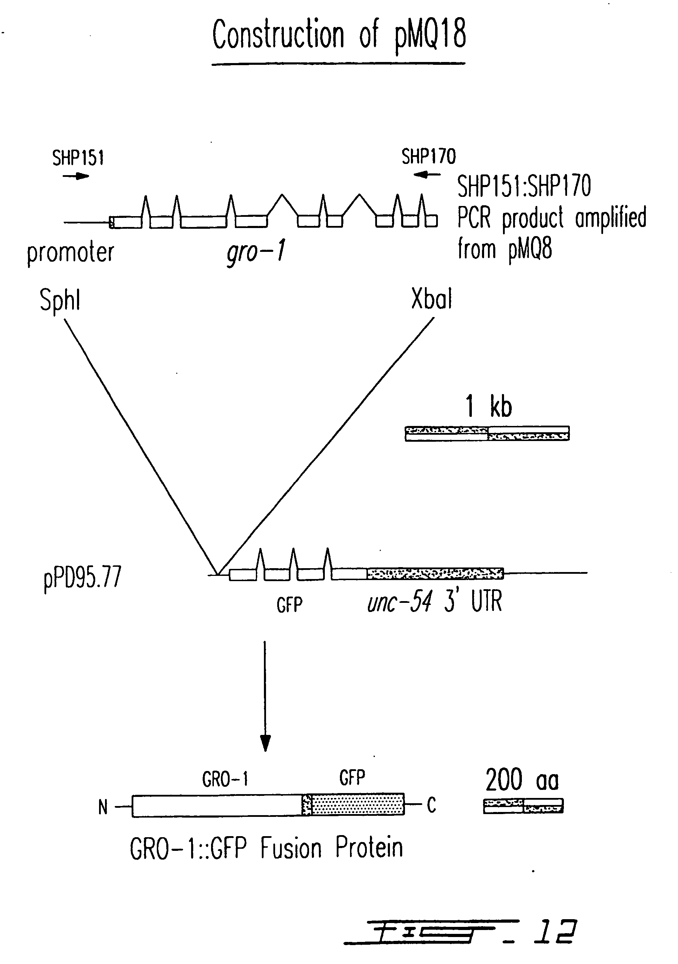

To establish the splicing pattern of this gene, cDNAs encompassing the 5' and 3' halves of the gene were produced by reverse transcription-PCR and sequenced (Fig. 3).

This revealed that the gene is composed of 9 exons, spans ~2 kb, and produces an mRNA of 1.3 kb. To confirm that this is indeed the gro-1 gene, genomic DNA was amplified by PCR from a strain containing the *gro-1(e2400)* mutation and the amplified product was sequenced. A lesion was found in the 5th exon, where a 9 base-pair sequence has been replaced by a 2 base-pair insertion, leading to a frameshift (Fig. 3C). Fig. 3C illustrates those residues which differ from wild type are in bold.

The reading frame continues out-of-frame for another 33 residues before terminating.

Figs. 3A-B illustrate the coding sequence in capital letters, while the introns, and the untranslated and intergenic sequence are in lower case letters. The protein sequence is shown underneath the coding sequence. Position 1 of the nucleotide sequence is the first base after the SL2 trans-splice acceptor sequence. Position 1 of the protein sequence is the initiator methionine. All PCR primers used for genomic and cDNA amplification are represented by arrows. For primers extending downstream (arrows pointing right) the primer sequence corresponds exactly to the nucleotides over which the arrow extends. But for primers extending upstream (arrows pointing left) the primer sequence is actually the complement of the sequence under the arrow. In both cases the arrow head is at the 3' end of the primer. The sequence of the two primers which flank *gro-1* (SHP93 and SHP92) are not represented in this figure. Their sequences are: SHP93 TTTCTGGATTTTAACCTTCC (SEQ. ID. N0:10) and SHP92 GATAGTTCCCTTCGTTCGGG (SEQ. ID. NO:9). The wild type splicing pattern was determined by sequencing of the cDNA. Identification of the *e2400* lesion was accomplished by sequencing the *e2400* allele. The *e2400* lesion consists of a 9 bp deletion and a 2 bp insertion at position 1196, resulting in a frameshift.

### gro-1 is part of a complex operon (Figs. 3A-3B)

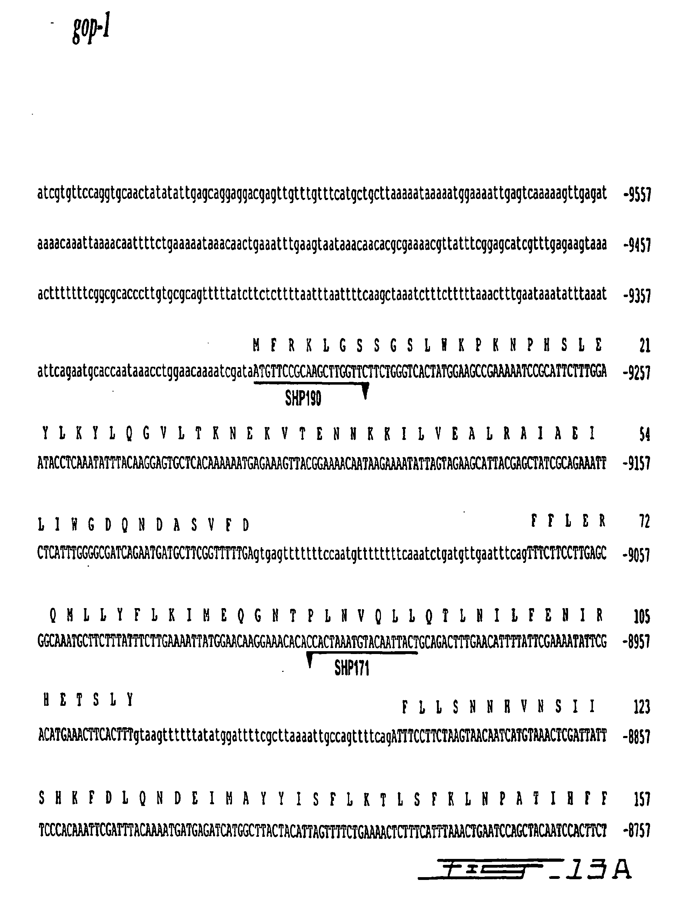

Amplification of the 5' end of *gro-1* from cDNA occurred only when the trans-spliced leader SL2 was used as the 5' primer, and not when SL1 was used. SL2 is used for trans-splicing to the downstream gene when two genes are organized into an operon (Spieth et al., Cell 73: 521-532 (1993); Zorio et al., Nature 372: 270-272 (1994)). This indicates that at least one gene upstream of *gro-1* is co-transcribed with *gro-1* from a common promoter. We found that sequences from the 5' end of the three next predicted genes upstream of gro-1 (ZC395.7, C34E10.1, and C34E10.2) all could only be amplified with SL2. Sequences from the fourth predicted upstream gene (C34E10.3), however, could be amplified with neither spliced leader, suggesting that it is not *trans*-spliced. The distance between genes in operons appear to have an upper limit (Spieth et al., Cell 73: 521-532 (1993); Zorio et al., Nature 372: 270-272 (1994)), and no gene is predicted to be close enough upstream of C34E10.3 or downstream of *gro*-*1* to be co-transcribed with these genes. Our findings suggest therefore that *gro-1* is the last gene in an operon of five co-transcribed genes (Fig. 4).

Nested PCR was used to amplify the 5' end of each gene. SL1 or SL2 specific primers were used in conjunction with a pair of gene-specific primers. cDNA generated by RT-PCR using mixed stage N2 RNA was used as template in the nested PCR. Fig. 4A illustrates a schematic of the *gro-1* operon showing the coding sequences of each gene and the primers (represented by flags) used to establish the trans-splicing patterns.

Fig. 4B illustrates the products of the PCR with SL1 and SL2 specific primers for each of the five genes. The sequences of the primers used are as follows: SL1: TTTAATTACCCAAGTTTGAG (SEQ. ID. NO:61), SL2: TTTTAACCCAGTTACTCAAG (SEQ. ID. NO:62), SHP141: AAAACTTCTACCAACAATGG (SEQ. ID. NO:30), SHP142: CGTAATCTCTCTCGATTAGC (SEQ. ID. NO:31), SHP143: CCGTGGGATGGCTACTTGCC (SEQ. ID. NO:32), SHP144: TGGATTTGTGGCACGAGCGG (SEQ. ID. NO:33), SHP145: TTGATTGCCTCTCCTCGTCC (SEQ. ID. NO:34), SHP146: ATCAACATCTGATTGATTCC (SEQ. ID. NO:35), SHP130: CATCCAAAAGGAGTATCACC (SEQ. ID. NO:24), SHP119: ACATCTTTATCCATTTCTCC (SEQ. ID. NO:21), SHP95: TACAGGAATTTTTGAACGGG (SEQ. ID. NO:12), SHP99: ATCGATACCACCGTCTCTGG (SEQ. ID. NO:16).

The gene immediately upstream of *gro-1,* has homology to the yeast gene *HAM1,* and we have renamed the gene *hap-1.* We have established its splicing pattern by reverse transcription PCR and sequencing. This revealed that *hap-1* is composed of 5 exons and produces an mRNA of 0.9 kb. We also found that sequences which were predicted to belong to ZC395.7 (now *hap-1)* are in fact spliced to the exons of C34E10.1. This is consistent with our finding that *hap-1* is SL2 spliced as it puts the end of the C34E10.1 very close to the start of *hap-1* (Fig. 4).

### The gro-1 gene product

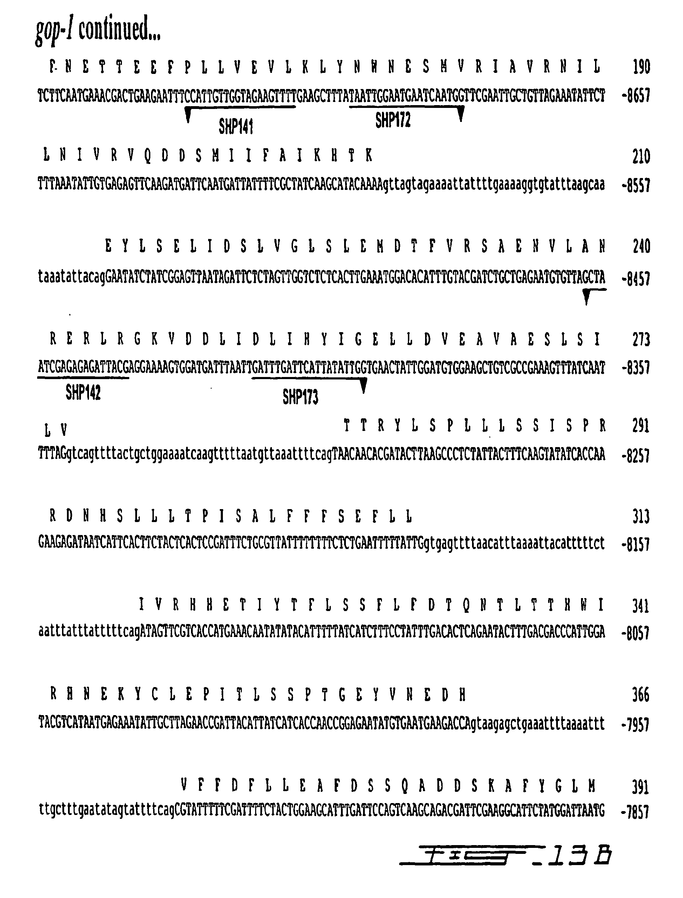

Conceptual translation of the *gro-1* transcript indicated that it encodes a protein of 430 amino acids highly similar to a strongly conserved cellular enzyme: dimethylallyldiphosphate:tRNA dimethylallyltransferase (DMAPP transferase). Fig. 5 shows an alignment of *gro-1* with the published sequences of the *E*. *coli* (P16384) and yeast (P07884) enzymes. Residues where the biochemical character of the amino acids is conserved are shown in bold. Identical amino acids are indicated further with a dot. The ATP/GTP binding site and the C2H2 zinc finger site are predicted and not experimental. The point at which the *gro-1(e2400)* mutation alters the reading frame of the sequence is shown. The two alternative initiator methionines in the yeast sequence, and the putative corresponding methionines in the worm sequence, are underlined.

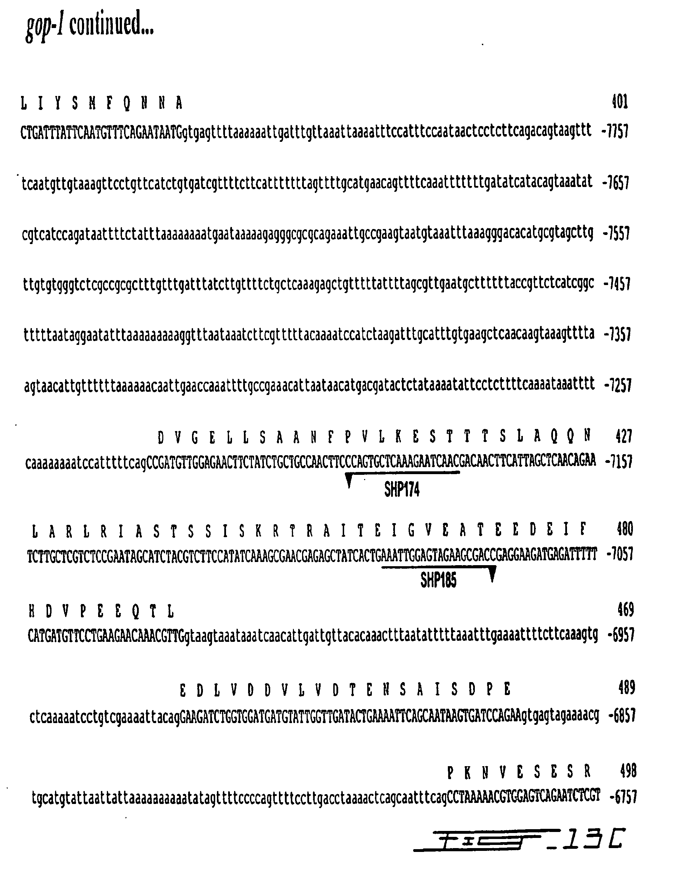

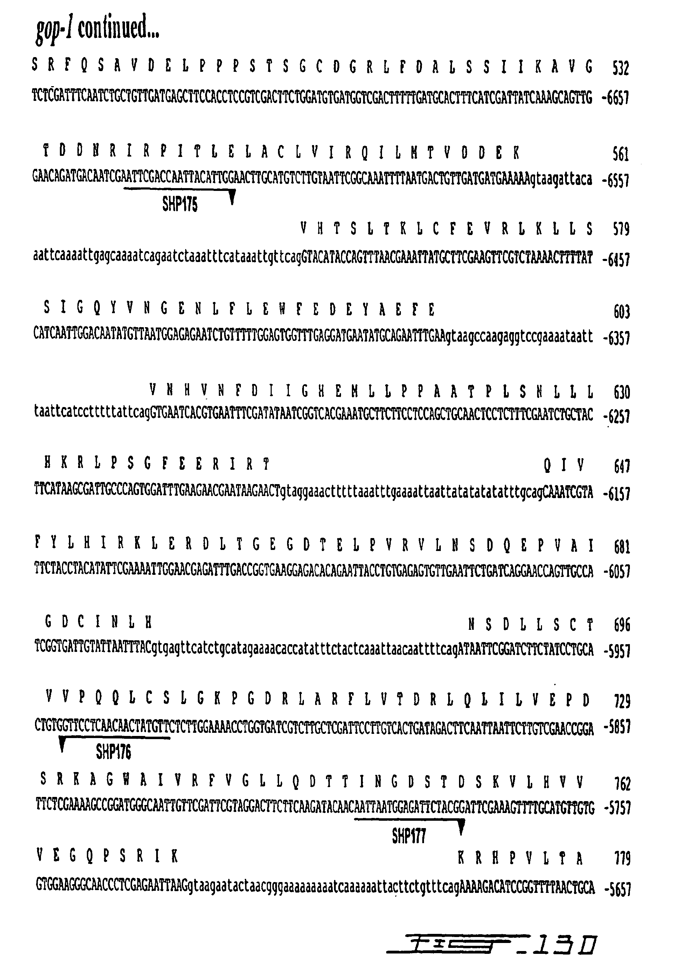

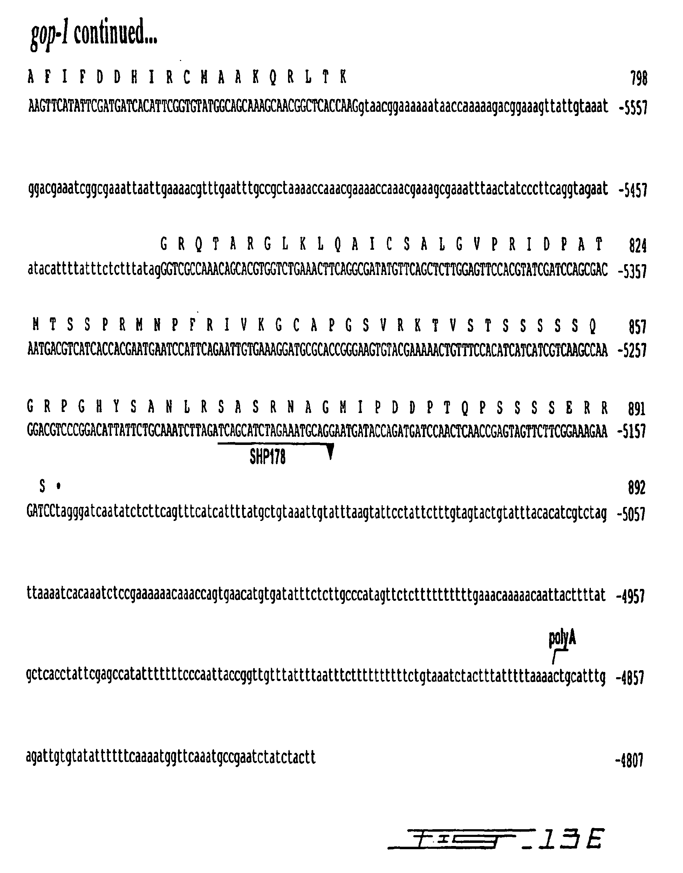

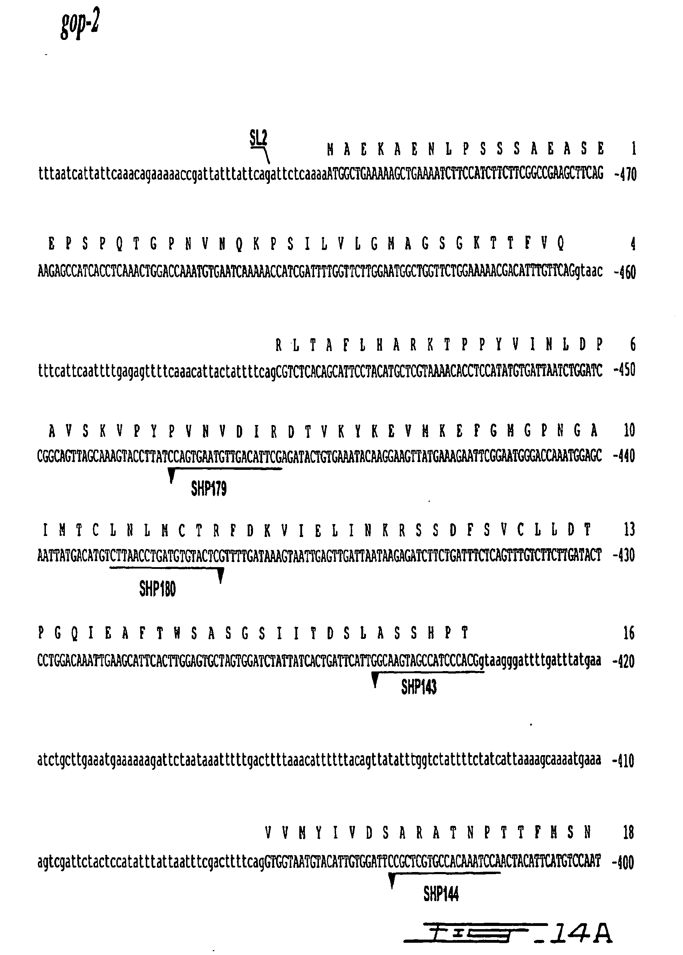

Database searches also identified a homologous human expressed sequence tag (Genbank ID: Z40724). The human clone has been used to derive a sequence tagged site (STS). This means that the genetic and physical position of the human *gro-1* homologue is known. It maps to chromosome 1, 122.8 cR from the top of Chr 1 linkage group and between the markers D1S255 and D1S2861. This information was found in the UniGene database or the National Center for Biotechnology Information (NCBI). We have sequenced Z40724 by classical methods but found that Z40724 is not a full length cDNA clone as it does not contain an initiator methionine nor the poly A tail. We used the sequence of Z40724 to identify further clones by database searches. We found one clone (Genbank ID: AA332152) which extended the sequence 5' by 28 nucleotides, as well as one clone (Genbank ID: AA121465) which extended the sequence substantially in the 3' direction but didn't include the poly A tail. We then used AA121465 to identify an additional clone (AA847885) extending the sequence to the poly A tail. Fig. 8 shows the full sequence with the putative initiator ATG shown in bold and the sequence of Z60724 is shown underlined. A comparison of the conceptual amino acid sequences for GRO-1 and hgro-1p is shown in Fig. 9. Amino acid identities are indicated by a dot. Both sequences contain a region with a zinc finger motif which is shown underlined.

An additional metazoan homologue is represented by Drosophila EST: Genbank accession: AA816785. In *E*. *coli* and other bacteria, the gene encoding DMAPP transferase is called *miaA* (a.k.a *trpX)* and is called *mod5* in yeast. DMAPP transferase catalyzes the modification of adenosine 37 of tRNAs whose anticodon begins with U (Fig. 6).

In these organisms the enzyme has been shown to use dimethylallyldiphosphate as a donor to generate dimethylallyl-adenosine (dma⁶A37), one base 3' to the anticodon (for review and biochemical characterization of the bacterial enzyme see Persson et al., Biochimie 76: 1152-1160 (1994); Leung et al., J Biol Chem 272: 13073-13083 (1997); Moore and Poulter, Biochemistry 36:604-614 (1997)). In earlier literature this modification is often referred to as isopentenyl adenosine (i⁶A37).

The high degree of conservation of the protein sequence between GRO-1 and DMAPP in *S*. *cerevisiae* and *E. coli* suggest that GRO-1 possesses the same enzymatic activity as the previously characterized genes. The sequence contains a number of conserved structural motifs (Fig. 5), including a region with an ATP/GTP binding motif which is generally referred to as the 'A' consensus sequence (Walker et al., EMBO J 1: 945-951 (1982)) or the 'P-loop' (Saraste et al., Trends Biochem Sci 15: 430-434 (1990)).

In addition, at the C-terminal end of the GRO-1 sequence, there is a C2H2 zinc finger motif as defined by the PROSITE database. This type of DNA-binding motif is believed to bind nucleic acids (Klug and Rhodes, Trends Biochem Sci 12: 464-469 (1987)). Although there appears to be some conservation between the worm and yeast sequences in the C-terminus end of the protein (Fig. 5), including in the region encompassing the zinc finger in GRO-1, the zinc finger motif per se is not conserved in yeast but is present in humans (Fig. 9).

In yeast DMAPP transferase is the product of the *MOD5* gene, and exists in two forms: one form which is targeted principally to the mitochondria, and one form which is found in the cytoplasm and nucleus. These two forms differ only by a short N-terminal sequence whose presence or absence is determined by differential translation initiation at two "in frame" ATG codons. (Gillman et al., Mol & Cell Biol 11: 2382-90 (1991)). The *gro*-1 open reading frame also contains two ATG codons at comparable positions, with the coding sequence between the two codons constituting a plausible mitochondrial sorting signal (Figs. 3 and 5). It is likely therefore that DMAPP transferase in worms also exists in two forms, mitochondrial and cytoplasmic.

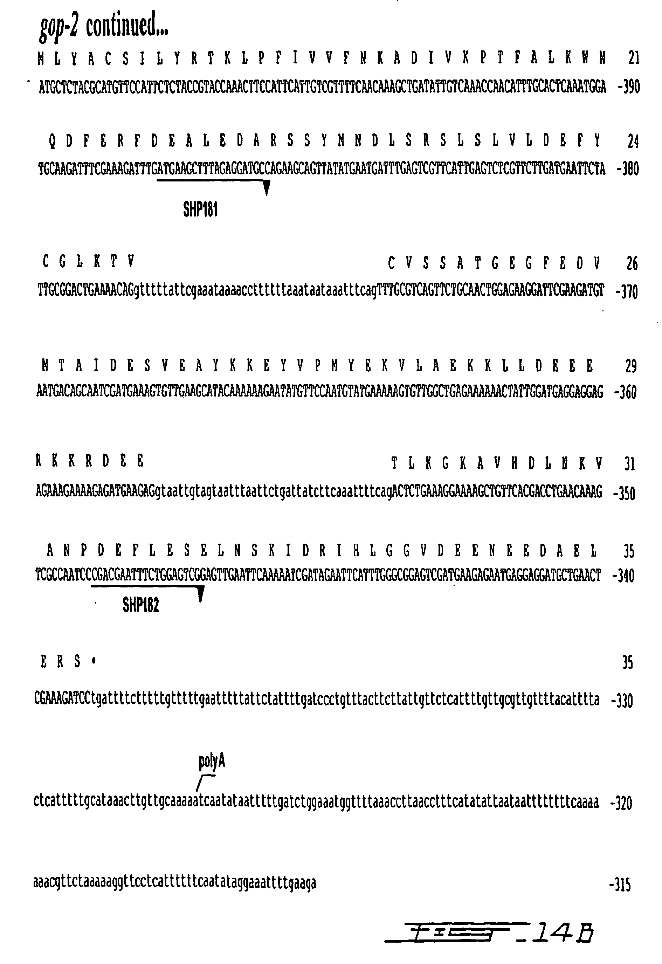

It should be noted, however, that the sequence of hgro-1 shows only one in-frame methionine before the conserved ATP/GTP binding site (Fig. 9). As we cannot be assured to have determined the sequence of the full length transcript, it is possible that further 5' sequence might reveal an additional methionine. Alternatively, in humans, the mechanism by which the enzyme is targeted to several compartments might not involved differential translation initiation. In this context, it should be noted that the sorting signals which can be predicted from the sequence of hgro-1p are predicted to be highly ambiguous by the prediction program PSORT II. Furthermore, a conceptual translation of the Drosophila sequence (AA816785) predicts only one initiator methionine before the ATP/GTP binding site as well as several in-frame stop codons upstream of this start (Fig. 10), suggesting that no additional upstream ATG could serve as translation initiation site. In the figure, stop codons are indicated by *stop*, methionines are indicated by **Met**, and the conserved ATP/GTP binding site is underlined.

### Expression pattern of GRO-1

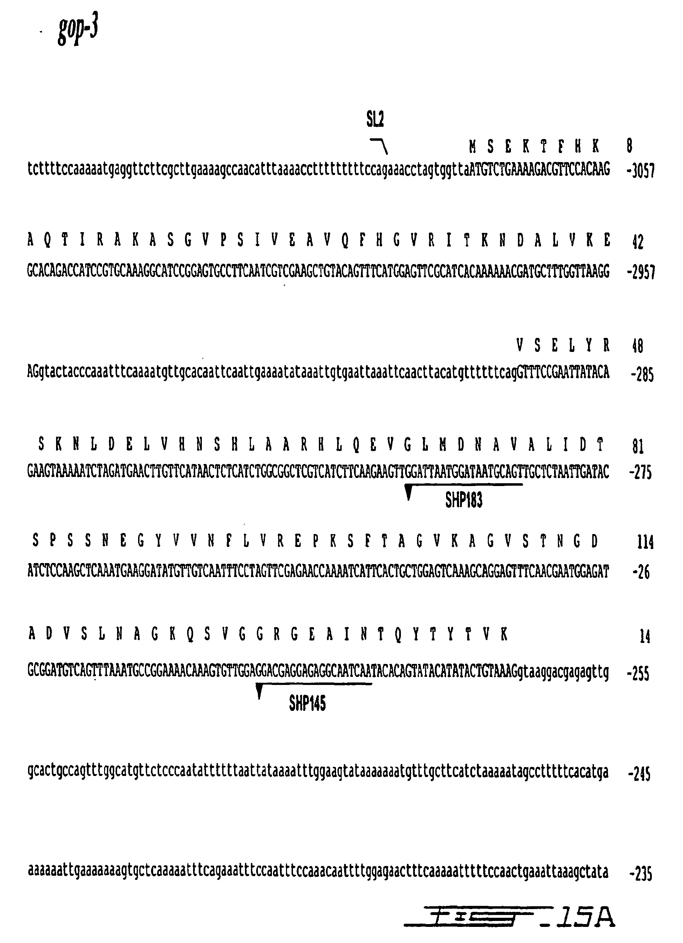

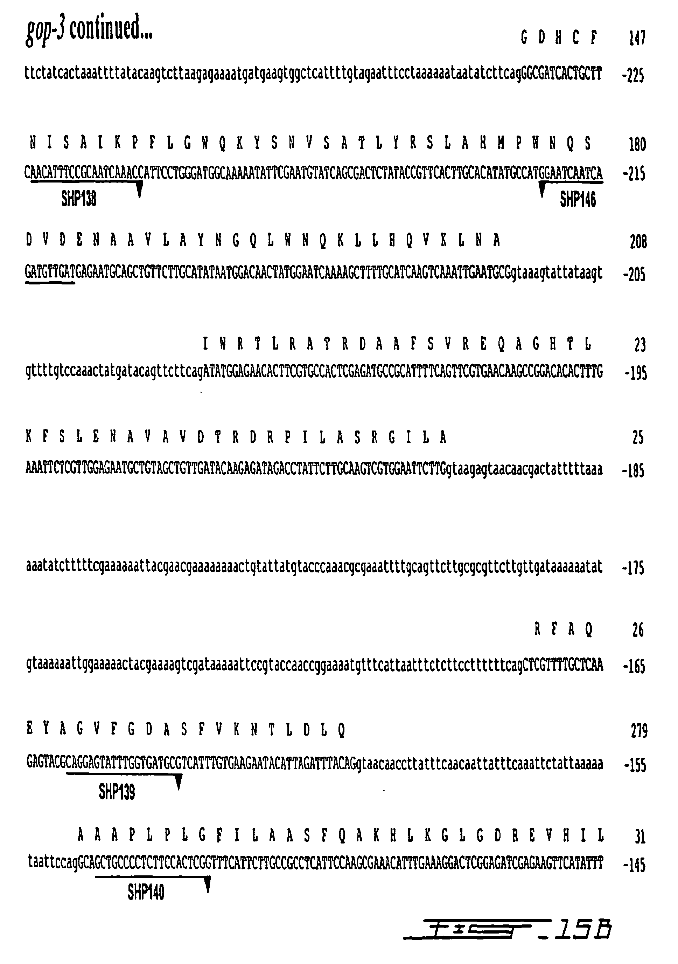

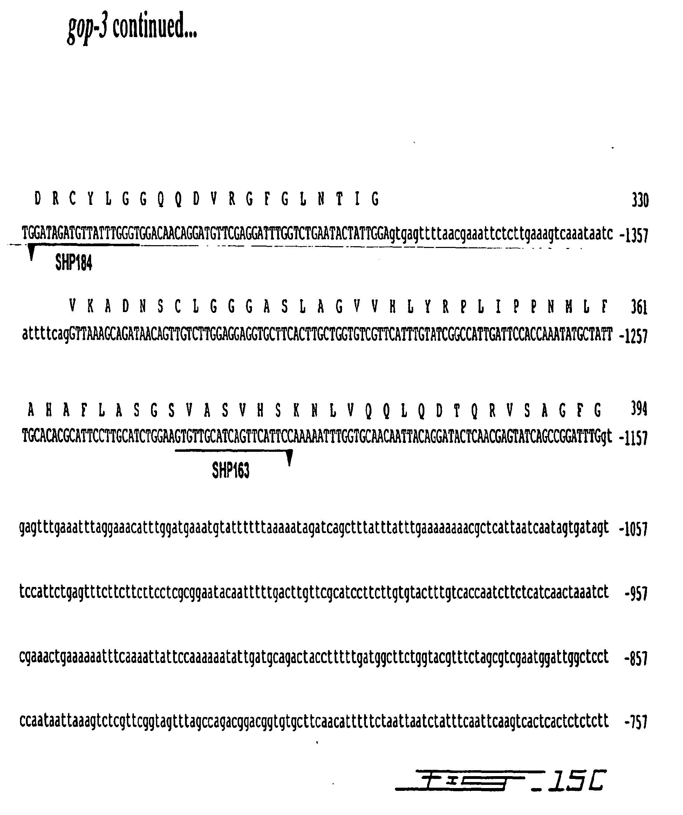

We have also constructed a reporter gene expressing a fusion protein containing the entire GRO-1 amino acid sequence fused at the C-terminal end to green fluorescent protein (GFP). The promotor of the reporter gene is the sequence upstream of *gop-1* (Figs. 13A-13C), the first gene in the operon (see Fig. 4). The promotor sequence is 306 bp long starting 32 nucleotides upstream of the *gop-1* ATG. It is fused at the exact level upstream of *gro-1* where trans-splicing to SL2 normally occurs.

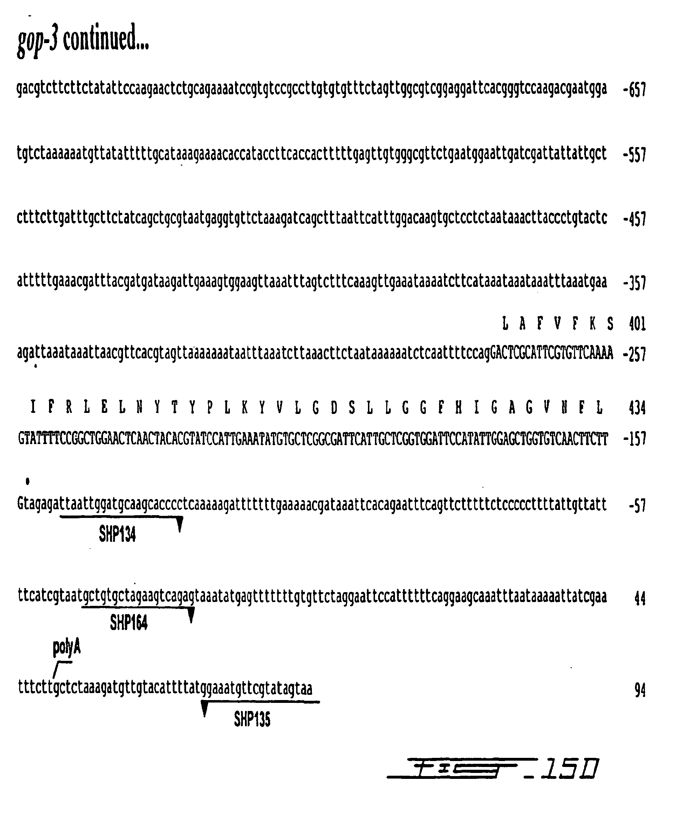

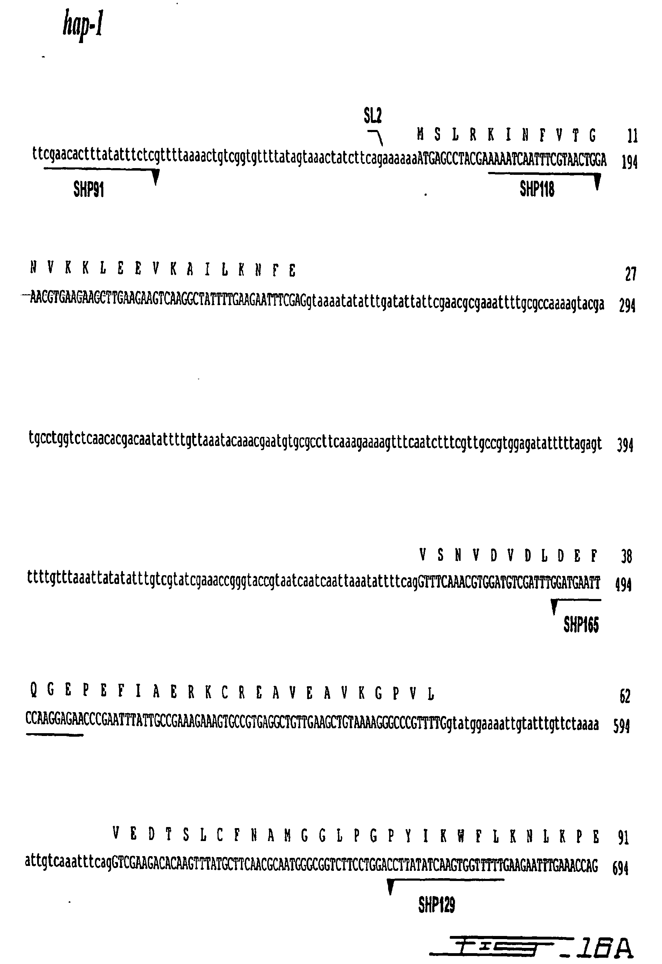

The genes *gop-2* (Fig. 14) and *gop-3* (Figs. 15A-15B) are also located in the operon (see Fig. 4), the second and third genes in the operon.

We first construct the clone pMQ8 in which *gro-1* is directly under the promoter for the whole operon using the hybrid primers SHP160 (SEQ. ID. NO:38) and SHP159 (SEQ. ID. NO:37) and the flanking primers SHP161 (SEQ. ID. NO:39) and SHP162 (SEQ. ID. NO:40) in sequential reactions each followed by purification of the products and finally cloning into pUC18 (Fig. 11).

Primers SHP151 (SEQ. ID. NO:36) and SHP170 (SEQ. ID. NO:44) where then used to amplify part of the insert in pMQ8 and clone in pPD95.77 (gift from Dr Andrew Fire) which was designed to allow a protein of interest to be transcriptionally fused to Green Fluorescent Protein (GFP) (Fig. 12).

The reporter construct fully rescues the phenotype of a *gro-1(e2400)* mutant upon injection and extrachromosomal array formation, indicating that the fusion to the GFP moiety does not significantly inhibit the function of GRO-1. Fluorescent microscopy indicated that *gro-1* is expressed in most or all somatic cells. Furthermore, the GRO-1::GFP fusion protein is localized in the mitochondria, in the cytoplasm as well as in the nucleus.

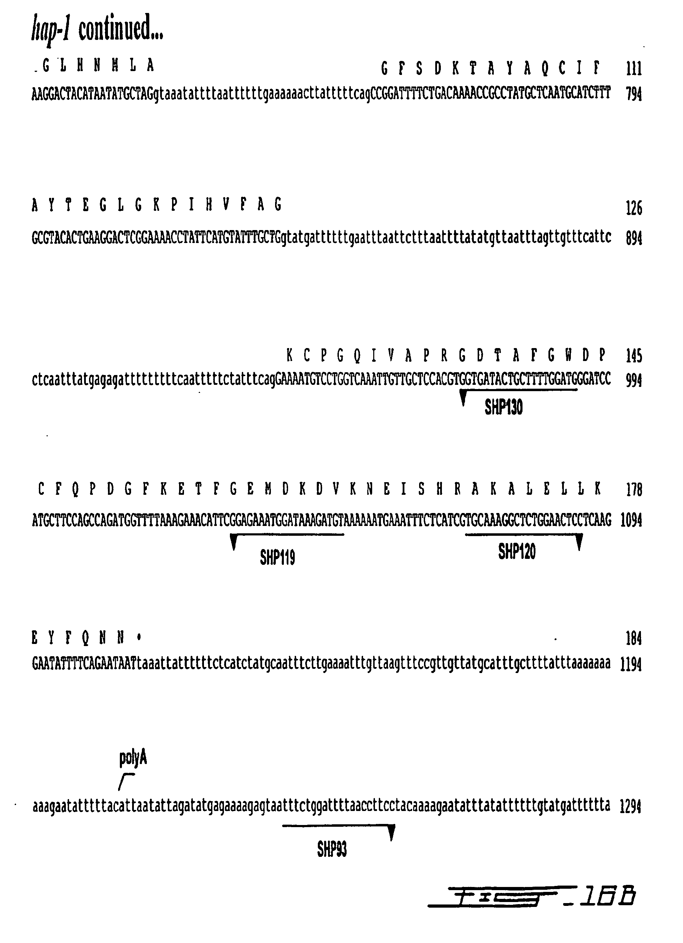

### The hap-1 gene product (Fig. 16)

*hap-1* is homologous to the yeast gene *HAM1* as well as to sequences in many organisms including bacteria and mammals (Fig. 7).

The origin of the worm and yeast sequence is as described above and below. The human sequence was inferred from a cDNA sequence assembled from expressed sequence tags (ESTs); the accession numbers of the sequences used were: AA024489, AA024794, AA025334, AA026396, AA026452, AA026502, AA026503, AA026611, AA026723, AA035035, AA035523, AA047591, AA047599, AA056452, AA115232, AA115352, AA129022, AA129023, AA159841, AA160353, AA204926, AA226949, AA227197 and D20115. The *E*. *coli* sequence is a predicted gene (accession 1723866).

Mutations in *HAM1* increase the sensitivity of yeast to the mutagenic compound 6-N-hydroxylaminopurine (HAP), but do not increase spontaneous mutation frequency (Nostov *et al., Yeast* **12**:17-29 (1996)). HAP is an analog of adenine and *in vitro* experiments suggest that the mechanism of HAP mutagenesis is its conversion to a deoxynucleoside triphosphate which is incorporated ambiguously for dATP and dGTP during DNA replication (Abdul-Masih and Bessman, J Biol Chem 261 (5): 2020-2026 (1986)). The role of the Ham1p gene product in increasing sensitivity to HAP remains unclear.

### Explaining the pleiotropy of miaA and gro-1

Mutations in *miaA*, the bacterial homologue of *gro-1,* show multiple phenotypes and affect cellular growth in complex ways. For example, in *Salmonella typhimurium*, such mutations result in 1) a decreased efficacy of suppression by some suppressor tRNA, 2) a slowing of ribosomal translation, 3) slow growth under various nutritional conditions, 4) altered regulation of several amino acid biosynthetic operons, 5) sensitivity to chemical oxidants and 6) temperature sensitivity for aerobic growth (Ericson and Björk, J. Bacteriol. 166: 1013-1021 (1986); Blum, J. Bacteriol. 170: 5125-5133 (1988)). Thus, MiaAp appears to be important in the regulation of multiple parallel processes of cellular physiology. Although we have not yet explored the cellular physiology of *gro-1* mutants along the lines which have been pursued in bacteria, the apparently central role of *miaA* is consistent with our findings that *gro-1,* and the other genes with a Clk phenotype, regulate many disparate physiological and metabolic processes in *C*. *elegans* (Wong et al., Genetics 139: 1247-1259 (1995) ; Lakowski and Hekimi, Science 272: 1010-1013 (1996); Ewbank et al., Science 275: 980-983 (1997)).

In addition to the various phenotypes discussed above, *miaA* mutations increase the frequency of spontaneous mutations (Connolly and Winkler, J Bacteriol 173(5): 1711-21 (1991); Connolly and Winkler, J Bacteriol 171: 3233-46 (1989)). As described in the previous section we have preliminary evidence that *gro-1(e2400)* also increases the frequency of spontaneous mutations in worms.

How can the alteration in the function of MDAPP transferase result in so many distinct phenotypes? Bacterial geneticists working with *miaA* have generally suggested that this enzyme and the tRNA modification it catalyzes have a regulatory function which is mediated through attenuation (e.g. Ericson and Björk, J. Bacteriol. 166: 1013-1021 (1986)). Attenuation is a phenomenon by which the transcription of a gene is interrupted depending on the rate at which ribosomes can translate the nascent transcript. Ribosomal translation is slowed in *miaA* mutants, and thus, through an effect on attenuation, could affect the expression of many genes whose expression is regulated by attenuation.

*gro*-*1*(*e*2400) also produces pleiotropic effects and, in addition, displays a maternal-effect, suggesting that it is involved in a regulatory process (Wong et al., Genetics 139: 1247-1259 (1995). However, attenuation involves the co-transcriptional translation of nascent transcripts, which is not possible in eukaryotic cells were transcription and translation are spatially separated by the nuclear membrane. If the basis of the pleiotropy in *miaA* and *gro-1* is the same, then a mechanism distinct from attenuation has to be involved. Below we argue that this mechanism could be the modification by DMAPP transferase of adenine residues in DNA in addition to modification of tRNAs.

### A role for gro-1 in DNA modification?

We observed that *gro-1* can be rescued by a maternal effect, so that adult worms homozygous for the mutation, but issued from mother carrying one wild type copy of the gene display a wild type phenotype, in spite of the fact that such adults are up to 1000 fold larger than the egg produced by their mother. It is unlikely that enough wild type product can be deposited by the mother in the egg to rescue a adult which is 1000 times larger. This observation suggests therefore that *gro-1* can induce an epigenetic state which is not altered by subsequent somatic growth. One of the best documented epigenetic mechanisms is imprinting in mammals (Lalande, Annu Rev Genet 30: 173-196 (1996)) which is believed to rely on the differential methylation of genes (Laird and Jaenisch, Annu Rev Genet 30: 441-464; Klein and Costa, Mutat Res 386: 103-105 (1997)). Modification of bases in DNA have also been linked to regulation of gene expression in the protozoan *Trypanosoma brucei.* The presence of beta-D-glucosyl-hydroxy-methyluracil in the long telomeric repeats of *T. brucei* correlates with the repression of surface antigen gene expression (Gommers-Ampt et al., Cell 75: 112-1136 (1993); van Leeuwen et al., Nucleic Acids Res 24: 2476-2482 (1996)).

*gro-1* and *miaA* increase the rate of spontaneous mutations, which is generally suggestive of a role in DNA metabolism, and can be related to the observation that methylation is linked to spontaneous mutagenesis, genome instability, and cancer (Jones and Gonzalgo, Proc. Natl. Acad. Sci. USA, 94: 2103-2105 (1997)).

Does *gro-1* have access to DNA? Studies with *mod5,* the yeast homologue of *gro-1,* have shown that there are two forms of Mod5p, one is localized to the nucleus as well as to the cytoplasm, and the other form is localized to the mitochondria as well as the cytoplasm (Boguta et al., Mol. Cell. Biol. 14: 2298-2306 (1994)). The nuclear localization is striking as isopentenylation of nuclear-encoded tRNA is believed to occur exclusively in the cytoplasm (reviewed in Boguta et al., Mol. Cell. Biol. 14: 2298-2306 (1994)). Furthermore, studies of a gene ma*f1* have shown that when *mod5* is mislocalized to the nucleus, the efficiency of certain suppressor tRNA is decreased, an effect known to be linked to the absence of the tRNA modification (Murawski et al., Acta Biochim. Pol. 41: 441-448 (1994)). Finally, as described in the previous section, *gro-1* contains a zinc finger, a nuclei acid binding motif. The zinc finger could bind tRNAs, but as it is in the C-terminal domain of *gro-1* and human hgro-1 that has no equivalent in *miaA,* it is clearly not necessary for the basic enzymatic function. We speculate that it might be necessary to increase the specificity of DNA binding in the large metazoan genome. It should also be noticed that the second form of Mod5p which is localized to mitochondria also has the opportunity to bind and possibly modify DNA as it has access to the mitochondrial genome. See the previous section entitled "A role for *gro-1* in a central mechanism of physiological coordination" for an alternative possibility as to the function of GRO-1 in the nucleus.

### miaA and gro-1 are found in complex operons

We have found that *gro-1* is part of a complex operon of five genes (Fig. 4). It is believed that genes are regulated coordinately by single promoters when they participate in a common function (Spieth et al., Cell 73: 521-532 (1993)). In some cases, this is well documented. For example, the proteins LIN-15A and LIN-15B which are both required for vulva formation in *C. elegans,* are unrelated products from two genes transcribed in a common operon (Huang et al., Mol Biol Cell 5(4): 395-411 (1994)). One of the genes in the *gro-1* promoter is *hap-1,* whose yeast homologue has been shown to be involved in the control of mutagenesis (Nostov et al., Yeast 12: 17-29 (1996)). Under the hypothesis that *gro-1* modifies DNA, it suggest an involvement of *hap-1* in this or similar processes. The presence in the same operon also suggest that all five genes might collaborate in a common function. The phenotype of *gro-1* suggests that this function is regulatory. In this context, it should be noted that *miaA* also is part of a particularly complex operon (Tsui and Winkler, Biochimie 76: 1168-1177 (1994)), although, except for *miaAlgro-1,* there are no other homologous genes in the two operons.

### A role for gro-1 in a central mechanism of physiological coordination

We have speculated that the genes with a Clk phenotype might participate in a central mechanism of physiological coordination, probably including the regulation of energy metabolism. *clk-1* encodes a mitochondrial protein (unpublished observations), and its homologue in yeast has also been shown to be mitochondrial (Jonassen, T (1998) Journal of Biological Chemistry 273:3351-3357). The yeast *clk-1* homologue is involved in the regulation of the biosynthesis of ubiquinone (Marbois, B.N. and Clarke, C.F. (1996) Journal of Biological Chemistry 271:2995-3004). Ubiquinone, also called coenzyme Q, is central to the production of ATP in mitochondria. In worms, however, we have found that *clk-1* is not strictly required for respiration. How might *gro-1* fit into this picture?

One link is that dimethylallyldiphosphate is known to be the precursor of the lipid side-chain of ubiquinone. In bacteria, ubiquinone is the major lipid made from DMAPP. In eukaryotes cholesterol and its derivatives are also made from DMAPP. Interestingly, *C. elegans* requires cholesterol in the growth medium for optimal growth. This link, however, remains tenuous, in particular in the absence of an understanding of the biochemical function of CLK-1.

In several bacteria, the adenosine modification carried out by DMAPP transferase is only the first step in a series of further modification of this base (Persson et al., Biochimie 76: 1152-1160 (1994)). These additional modifications have been proposed to play the role of a sensor for the metabolic state of the cell (Buck and Ames, Cell 36: 523-531 (1984); Persson and Björk, J. Bacteriol. 175: 7776-7785 (1993)). For example, one of the subsequent steps, the synthesis of 2-methylthio-cis-ribozeatin is carried out by a hydroxylase encoded by the gene *miaE.* When the cells lack miaE they become incapable of using intermediates of the citric acid cycle such as fumarate and malate as the sole carbon source.

Another link to energy metabolism springs from the recent biochemical observations of Winkler and coworkers using purified DMAPP transferase (*E. coli* MiaAp) (Leung et al., J Biol Chem 272: 13073-13083 (1997)). These investigators observed that the enzyme in competitively inhibited by phosphate nucleotides such as ATP or GTP. Furthermore, using their estimation of *K*ₘ of the enzyme and its concentration in the cell, they calculate that the level of inhibition of the enzyme *in vivo,* would exactly allow the enzyme to modify all tRNAs but any further inhibition would leave unmodified tRNAs. This suggests that the exact level of modification of tRNA (or of DNA) could be exquisitely sensitive to the level of phosphate nucleotides. Superficially, this is consistent with the phenotypic observations. The state of mutant cells which lack DMAPP transferase entirely would be equivalent of cells where very high levels of ATP would completely inhibit the enzyme. Such cells might therefore turn down the ATP generating processes in response to the signal provided by undermodified tRNAs (or DNA).

More generally, GRO-1 could act in the crosstalk between nuclear and mitochondrial genomes. The nuclear and mitochondrial genomes both contribute gene products to the mitochondrion energy-producing machinery and these physically separate genomes must therefore exchange information somehow to coordinate their contributions (reviewed in Poyton, R.O. and McEwen J.E. (1996) Annu. Rev. Biochem. 65:563-607). Furthermore, the energy producing activity of the mitochondria is essential to the rest of the cell, and the needs of a particular cell at a particular time must be somehow convey to the organelle to regulate its activity. GRO-1 could participate in this coordination in the following manner. GRO-1 is found in three compartments, the nucleus, the cytoplasm and the mitochondria (see above), and thus has the opportunity to regulate gene expression in more that one way. How could its action coordinate gene expression between compartment? GRO-1 could partition between the mitochondria and the nucleus and its relative distribution could be determined by the amount of RNA (or mtDNA) in the mitochondria (Parikh, V.S. et al. (1987) Science 235:576-580). For example, if the cell is rich in mitochondria, much GRO-1 will be bound there which could result in a relative depletion of activity in the cytoplasm with regulatory consequences on the translation machinery. Binding of GRO-1 in the nucleus could have similar consequences and provide information about nuclear gene expression to the translation machinery.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. An isolated polynucleotide that encodes a polypeptide having the amino acid sequence encoded by the nucleotide sequence of SEQ ID N0:3 or a complement of said polynucleotide.

2. The isolated polynucleotide of claim 1 having a nucleotide sequence of SEQ ID NO:3 or a complement thereof

3. A fragment of the polypeptide encoded by the isolated polypeptide of claim 1 wherein said fragment can rescue at least one phenotype in a *gro-1*(e2400) *C. elegans* mutant.

4. The polynucleotide of claim 1 or 2 further comprising one or more nucleotide sequences encoding a heterologous polypeptide,

5. The polynucleotide of claim 4 wherein the heterologous polypeptide is green fluorescent protein (GFP).

6. A *gro-1* protein encoded by the polynucleotide of claim 1 or 2.

7. A *gop-3* gene which codes for a *gop-3* protein having the amino acid sequence set forth in SBQ ID NO:6; wherein said *gop-3* gene is located in the *gro-1* operon and said gop-3 gene is transcriptionally co-expressed with *gro-1* gene present in said operon.

8. A *hap-1* gene which codes for a *hap-1* protein having the amino acid sequence set forth in SEQ ID N0:7; wherein said *hap-1* gene is located in the *gro-1* operon and said *hap-1* gene is transcriptionally co-expressed with gro-1 gene present in said operon.

9. A mutant *gro-1* protein comprising the amino acid sequence of HAEYINHSKYGVTLVLKNSFHGSIWTHQKWIHSMGINCSSKDAMM.

10. A *gro-1* protein having the amino acid sequence of SEQ ID NO:2.

11. A *gop-3* protein encoded by the gene according to claim 7, wherein said protein has the amino acid sequence set forth in SEQ ID NO:6.

12. A *hap-1* protein encoded by the gene according to claim 8, wherein said protein has the amino acid sequence set forth in SEQ ID NO:7.

13. A method for the diagnosis and/or prognosis of cancer in a sample from a patient, which comprises analyzing DNA of said patient in said sample to determine if the human *gro-1* gene sequence is altered in comparison to the human *gro-1* nucleotide sequence set forth in SEQ ID NO:3, wherein alteration of the human *gro-1* gene is indicative of cancer.

14. A mouse, which comprises a gene knock-out of the murine *gro-1* gene that is homologous to the polypeptide of claim 1 or 2.

15. The use of a phosphate nucleotide for the manufacture of a medicament for interfering with enzymatic activity of *gro-1* of claim 6 or 10.

16. A vector comprising the polynucleotide of claim 1, 2, 4, or 5.

17. A vector comprising the gene of claim 7 or 8.

18. The vector of claim 16 or 17 that is an expression vector.

19. A host cell which comprises the vector of claim 16 or 17.

## Patentansprüche

1. Isoliertes Polynucleotid, das ein Polypeptid mit der Aminosäuresequenz, codiert durch die Nucleotidsequenz von SEQ ID NO:3, codiert oder ein Komplement des Polynucleotids.

2. Isoliertes Polynucleotid nach Anspruch 1 mit einer Nucleotidsequenz von SEQ ID NO:3 oder einem Komplement davon.

3. Fragment des Polypeptids, codiert durch das isolierte Polypeptid von Anspruch 1, wobei das Fragment mindestens einen Phänotyp in einer *gro-1 (e2400)-C.-elegans-*Mutante retten kann.

4. Polynucleotid nach Anspruch 1 oder 2, weiterhin umfassend eine oder mehrere Nucleotidsequenzen, codierend ein heterologes Polypeptid.

5. Polynucleotid nach Anspruch 4, wobei das heterologe Polypeptid grün fluoreszierendes Protein (GFP) ist.

6. *Gro-1-*Protein*,* codiert durch das Polynucleotid nach Anspruch 1 oder 2.

7. *Gop-3-*Gen*,* welches für ein gop-3-Protein mit der Aminosäuresequenz, angegeben in SEQ ID NO:6, codiert; wobei das *gop-3*-Gen sich in dem *gro-1*-Operon befindet und das *gop-3*-Gen transkriptionell mit dem *gro-1*-Gen, vorhanden in dem Operon, coexprimiert wird.

8. *Hap-1*-Gen, welches für ein *hap-1*-Protein mit der Aminosäuresequenz, angegeben in SEQ ID NO:7, codiert; wobei sich das *hap-1*-Gen in dem gro-1-Operon befindet und das *hap-1*-Gen transkriptionell mit dem *gro-1*-Gen, vorhanden in dem Operon, coexprimiert wird.

9. Mutantes *gro-1-Protein,* umfassend die Aminosäuresequenz von HAEYINHSKYGVTLVLKNSFHGSIWTHQKWIHSMGINCSSKDAMM.

10. *Gro-1*-Protein mit der Aminosäuresequenz von SEQ ID NO:2.

11. *Gop-3*-Protein, codiert durch das Gen gemäß Anspruch 7, wobei das Protein die Aminosäuresequenz, angegeben in SEQ ID NO:6, hat.

12. *Hap-1*-Protein, codiert durch das Gen gemäß Anspruch 8, wobei das Protein die Aminosäuresequenz, angegeben in SEQ ID NO:7, hat.

13. Verfahren für die Diagnose und/oder Prognose von Krebs in einer Probe von einem Patienten, welches Analysieren von DNA des Patienten in der Probe umfasst, um zu bestimmen, ob die humane *gro-1-*Gensequenz im Vergleich zu der humanen *gro-1-*Nucleotidsequenz, angegeben in SEQ ID NO:3, verändert ist, wobei Veränderung des humanen *gro-1-Gens* ein Anzeichen von Krebs ist.

14. Maus, welche ein Gen-Knockout des murinen *gro-1-*Gens umfasst, das homolog zu dem Polypeptid von Anspruch 1 oder 2 ist.

15. Verwendung eines Phosphatnucleotids für die Herstellung eines Medikaments zum Beeinträchtigen der enzymatischen Aktivität von *gro-1* nach Anspruch 6 oder 10.

16. Vektor, umfassend das Polynucleotid nach Anspruch 1, 2, 4 oder 5.

17. Vektor, umfassend das Gen nach Anspruch 7 oder 8.

18. Vektor nach Anspruch 16 oder 17, der ein Expressionsvektor ist.

19. Wirtszelle, welche den Vektor nach Anspruch 16 oder 17 umfasst.

## Revendications

1. Un polynucléotide isolé qui code pour un polypeptide ayant une séquence d'acides aminés codée par la séquence nucléotidique SEQ ID NO: 3 ou une séquence complémentaire dudit polynucléotide.

2. Le polynucléotide isolé de la revendication 1 ayant une séquence nucléotidique selon la SEQ ID NO: 3 ou une séquence complémentaire de celle-ci.

3. Un fragment de polynucléotide codé par le polypeptide isolé de la revendication 1 dans lequel ledit fragment peut recouvrer au moins un phénotype dans un mutant *C*. *elegans gro-1* (e2400).

4. Le polynucléotide de la revendication 1 ou 2 comprenant en outre une ou plusieurs séquences nucléotidiques qui codent pour un polypeptide hétérologue.

5. Le polynucléotide de la revendication 4 dans lequel le polypeptide hétérologue est la protéine verte fluorescente (GFP).

6. Une protéine *gro-1* codée par le polynucléotide de la revendication 1 ou 2.

7. Un gène *gop-3* qui code pour une protéine *gop-3* ayant une séquence d'acides aminés selon la SEQ ID NO: 6; dans lequel ledit gène *gop-3* est situé dans l'opéron *gro-1* et ledit gène *gop-3* est transcriptionnellement co-exprimé avec le gène *gro-1* présent dans ledit opéron.

8. Un gène *hap-1* qui code pour une protéine *hap-1* ayant une séquence d'acides aminés selon la SEQ ID NO: 7; dans lequel ledit gène *hap-1* est situé dans l'opéron *gro-1* et ledit gène *hap-1* est transcriptionnellement co-exprimé avec le gène *gro-1* présent dans ledit opéron.

9. Une protéine *gro-1* mutante comprenant la séquence d'acides aminés HAEYINHSKYGVTLVLKNSFHGSIWTHQKWIHSMGINCSSKDAMM.

10. Une protéine *gro-1* comprenant la séquence d'acides aminés SEQ ID NO: 2.

11. Une protéine *gop-3* codée par le gène selon la revendication 7, dans laquelle ladite protéine a une séquence d'acides aminés selon la SEQ ID NO: 6.

12. Une protéine *hap-1* codée par le gène selon la revendication 8, dans laquelle ladite protéine a une séquence d'acides aminés selon la SEQ ID NO: 7.

13. Une méthode pour le diagnostic et/ou le pronostic du cancer à partir d'un échantillon d'un patient, laquelle comprend l'analyse de l'ADN dudit patient dans ledit échantillon pour déterminer si la séquence du gène *gro-1* humain est altérée en comparaison de la séquence nucléotidique *gro-1* humaine selon la SEQ ID NO: 3, dans laquelle l'altération du gène *gro-1* humain est indicatif d'un cancer.

14. Une souris, laquelle comprend un gène knock-out d'un gène *gro-1* murin qui est homologue au polypeptide de la revendication 1 ou 2.

15. Utilisation d'un nucléotide phosphaté pour la fabrication d'un médicament pour interférer avec l'activité enzymatique de *gro-1* selon la revendication 6 ou 10.

16. Un vecteur comprenant le polynucléotide de la revendication 1, 2, 4 ou 5.

17. Un vecteur comprenant le gène de la revendication 7 ou 8.

18. Le vecteur selon la revendication 16 ou 17 est un vecteur d'expression.

19. Une cellule hôte, laquelle comprend un vecteur selon la revendication 16 ou 17.


### SEQUENCE LISTING

(1) GENERAL INFORMATION:
   (i) APPLICANT: McGILL UNIVERSITY
   (ii) TITLE OF INVENTION: THE C. ELEGANS gro-1 GENE
   (iii) NUMBER OF SEQUENCES: 62
   (iv) CORRESPONDENCE ADDRESS:
      (A) ADDRESSEE: SWABEY OGILVY RENAULT
      (B) STREET: 1981 McGill College Avenue - Suite 1600
      (C) CITY: Montréal
      (D) STATE: QC
      (E) COUNTRY: Canada
      (F) ZIP: H3A 2Y3
   (v) COMPUTER READABLE FORM:
      (A) MEDIUM TYPE: Diskette
      (B) COMPUTER: IBM Compatible
      (C) OPERATING SYSTEM: Windows
      (D) SOFTWARE: FastSEQ for Windows Version 2.0b
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: PCT/CA98/00803
      (B) FILING DATE: 20-AUG-1998
   (vii) PRIOR APPLICATION DATA:
      (A) APPLICATION NUMBER: CA 2,210,251
      (B) FILING DATE: 25-AUG-1997
   (viii) ATTORNEY/AGENT INFORMATION:
      (A) NAME: Côté, France
      (B) REGISTRATION NUMBER: 4166
      (C) REFERENCE/DOCKET NUMBER: 1770-179"EP" FC/gc
   (ix) TELECOMMUNICATION INFORMATION:
      (A) TELEPHONE: 514 845-7126
      (B) TELEFAX: 514 288-8389
      (C) TELEX:
(2) INFORMATION FOR SEQ ID N0:1:
   (i) SEQUENCE CHARACTERISTICS:
      (A) LENGTH: 14458 base pairs
      (B) TYPE: nucleic acid
      (C) STRANDEDNESS: single
      (D) TOPOLOGY: linear
   (ii) MOLECULE TYPE: Genomic DNA
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 430 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:2:
   Met Ile Phe Arg Lys Phe Leu Asn Phe Leu Lys Pro Tyr Lys Met Arg
    1               5                  10                  15
   Thr Asp Pro Ile Ile Phe Val Ile Gly Cys Thr Gly Thr Gly Lys Ser
               20                  25                  30
   Asp Leu Gly Val Ala Ile Ala Lys Lys Tyr Gly Gly Glu Val Ile Ser
           35                  40                  45
   Val Asp Ser Met Gln Phe Tyr Lys Gly Leu Asp Ile Ala Thr Asn Lys
       50                  55                  60
   Ile Thr Glu Glu Glu Ser Glu Gly Ile Gln His His Met Met Ser Phe
   65                  70                  75                  80
   Leu Asn Pro Ser Glu Ser Ser Ser Tyr Asn Val His Ser Phe Arg Glu
                   85                  90                  95
   Val Thr Leu Asp Leu Ile Lys Lys Ile Arg Ala Arg Ser Lys Ile Pro
               100                 105                 110
   Val Ile Val Gly Gly Thr Thr Tyr Tyr Ala Glu Ser Val Leu Tyr Glu
           115                 120                 125
   Asn Asn Leu Ile Glu Thr Asn Thr Ser Asp Asp Val Asp Ser Lys Ser
       130                 135                 140
   Arg Thr Ser Ser Glu Ser Ser Ser Glu Asp Thr Glu Glu Gly Ile Ser
   145                 150                 155                 160
   Asn Gln Glu Leu Trp Asp Glu Leu Lys Lys Ile Asp Glu Lys Ser Ala
                           165                     170                     175
   Leu Leu Leu His Pro Asn Asn Arg Tyr Arg Val Gln Arg Ala Leu Gln
                   180                     185                     190
   Ile Phe Arg Glu Thr Gly Ile Arg Lys Ser Glu Leu Val Glu Lys Gln
               195                     200                     205
   Lys Ser Asp Glu Thr Val Asp Leu Gly Gly Arg Leu Arg Phe Asp Asn
       210                     215                     220
   Ser Leu Val Ile Phe Met Asp Ala Thr Pro Glu Val Leu Glu Glu Arg
   225                     230                     235                     240
   Leu Asp Gly Arg Val Asp Lys Met Ile Lys Leu Gly Leu Lys Asn Glu
                   245                     250                     255
   Leu Ile Glu Phe Tyr Asn Glu His Ala Glu Tyr Ile Asn His Ser Lys
               260                     265                     270
   Tyr Gly Val Met Gln Cys Ile Gly Leu Lys Glu Phe Val Pro Trp Leu
           275                     280                     285
   Asn Leu Asp Pro Ser Glu Arg Asp Thr Leu Asn Gly Asp Lys Leu Phe
       290                     295                     300
   Lys Gln Gly Cys Asp Asp Val Lys Leu His Thr Arg Gln Tyr Ala Arg
   305                     310                     315                     320
   Arg Gln Arg Arg Trp Tyr Arg Ser Arg Leu Leu Lys Arg Ser Asp Gly
                   325                     330                     335
   Asp Arg Lys Met Ala Ser Thr Lys Met Leu Asp Thr Ser Asp Lys Tyr
               340                     345                     350
   Arg Ile Ile Ser Asp Gly Met Asp Ile Val Asp Gln Trp Met Asn Gly
           355                     360                     365
   Ile Asp Leu Phe Glu Asp Ile Ser Thr Asp Thr Asn Pro Ile Leu Lys
       370                     375                     380
   Gly Ser Asp Ala Asn Ile Leu Leu Asn Cys Glu Ile Cys Asn Ile Ser
   385                     390                     395                     400
   Met Thr Gly Lys Asp Asn Trp Gln Lys His Ile Asp Gly Lys Lys His
                   405                     410                     415
   Lys His His Ala Lys Gln Lys Lys Leu Ala Glu Thr Arg Thr
               420                     425                     430
(2) INFORMATION FOR SEQ ID NO:3:
   (i) SEQUENCE CHARACTERISTICS:
      (A) LENGTH: 2041 base pairs
      (B) TYPE: nucleic acid
      (C) STRANDEDNESS: single
      (D) TOPOLOGY: linear
   (ii) MOLECULE TYPE: Genomic DNA
   (xi) SEQUENCE DESCRIPTION: SEQ ID NO:3:
   CTGCCATAAG ATGGCGTCCG TGGCGGCTGC ACGAGCAGTT CCTGTGGGCA GTGGGCTCAG    60
   GGGCCTGCAA CGGACCCTAC CTCTTGTAGT GATTCTCGGG GCCACGGGCA CCGGCAAATC   120
   CACGCTGGCG TTGCAGCTAG GCCAGCGGCT CGGCGGTGAG ATCGTCAGCG CTGACTCCAT   180
   GCAGGTCTAT GAAGGCCTAG ACATCATCAC CAACAAGGTT TCTGCCCAAG AGCAGAGAAT   240
   CTGCCGGCAC CACATGATCA GCTTTGTGGA TCCTCTTGTG ACCAATTACA CAGTGGTGGA   300
   CTTCAGAAAT AGAGCAACTG CTCTGATTGA AGATATATTT GCCCGAGACA AAATTCCTAT   360
   TGTTGTGGGA GGAACCAATT ATTACATTGA ATCTCTGCTC TGGAAAGTTC TTGTCAATAC   420
   CAAGCCCCAG GAGATGGGCA CTGAGAAAGT GATTGACCGA AAAGTGGAGC TTGAAAAGGA   480
   GGATGGTCTT GTACTTCACA AACGCCTAAG CCAGGTGGAC CCAGAAATGG CTGCCAAGCT   540
   GCATCCACAT GACAAACGCA AAGTGGCCAG GAGCTTGCAA GTTTTTGAAG AAACAGGAAT    600
   CTCTCATAGT GAATTTCTCC ATCGTCAACA TACGGAAGAA GGTGGTGGTC CCCTTGGAGG    660
   TCCTCTGAAG TTCTCTAACC CTTGCATCCT TTGGCTTCAT GCTGACCAGG CAGTTCTAGA    720
   TGAGCGCTTG GATAAGAGGG TGGATGACAT GCTTGCTGCT GGGCTCTTGG AGGAACTAAG    780
   AGATTTTCAC AGACGCTATA ATCAGAAGAA TGTTTCGGAA AATAGCCAGG ACTATCAACA    840
   TGGTATCTTC CAATCAATTG GCTTCAAGGA ATTTCACGAG TACCTGATCA CTGAGGGAAA    900
   ATGCACACTG GAGACTAGTA ACCAGCTTCT AAAGAAAGGA CCTGGTCCCA TTGTCCCCCC    960
   TGTCTATGGC TTAGAGGTAT CTGATGTCTC GAAGTGGGAG GAGTCTGTTC TTGAACCTGC   1020
   TCTTGAAATC GTGCAAAGTT TCATCCAGGG CCACAAGCCT ACAGCCACTC CAATAAAGAT   1080
   GCCATACAAT GAAGCTGAGA ACAAGAGAAG TTATCACCTG TGTGACCTCT GTGATCGAAT   1140
   CATCATTGGG GATCGCGAAT GGGCAGCGCA CATAAAATCC AAATCCCACT TGAACCAACT   1200
   GAAGAAAAGA AGAAGATTGG ACTCAGATGC TGTCAACACC ATAGAAAGTC AGAGTGTTTC   1260
   CCCAGACTAT AACAAAGAAC CTAAAGGGAA GGGATCCCCA GGGCAGAATG ATCAAGAGCT   1320
   GAAATGCAGC GTTTAAGAGA CATGTCCAGT GGCCTTTGGA AAGGTGGTGG GGATCCAGTT   1380
   CAGGAGGGAG GGGTATGTTT GTCTCCCAGT CTGGGCAAAG GAGTGCTATG CGGAATTCTC   1440
   TGCATAGCAG AAAAGCTCCC ACCATTTTCT TTTGATGTGG TTTTAAAGTC TCACGTTCTC   1500
   TATAATAGAA ACAGCAGGTC TTGTCAGCTC CTTGTGTGGC TGATGTGTCT GGAAATGATG   1560
   TAGTTCAGGA AAGCATTTTT TTTTTCTTTG AACCTTAAAG GTTCTATTAT TAAAAGCAGC   1620
   ACAGATTCCA CATTTTTATA CATGAGGATC TTCTTTGTGG TGAATACCAG GATTGACTGC   1680
   ATCCCTTTAA AAGAAGTTTT ATGTCCCTGA CTCTGGCTAA AATTATCTAA TTTCCAGATG   1740
   CTTTTGTAGA TGACTGAAGT ATTTGTGAGC CACATATTGG GAGTTCTAGA TTTGAGTGAA   1800
   TGGCAGGAAA GGGCCATCTC CATTGAGATG ATTAAGTGAA CCAAACTAGT TCTCGGAATT   1860
   CTACAGAGAA GGAGGGAATC AGACTGAGGA AGCTGTGACA TAGGACTTGA AGACCAAAGA   1920
   CTTTGAAATT TGCGAGCTGC TCATGTGTGA GTTATTATCA CTGCTGTCTT TCTATTGAGT   1980
   TACAAATCTA TATTTTTATT GAAGTTTAAA TAAAGAAAAA ATTTACAAGA AAAAAAAAAA   2040
   A                                                                   2041
(2) INFORMATION FOR SEQ ID NO:4:
   (i) SEQUENCE CHARACTERISTICS:
      (A) LENGTH: 892 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID N0:4:
(2) INFORMATION FOR SEQ ID NO:5:
   (i) SEQUENCE CHARACTERISTICS:
      (A) LENGTH: 355 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:5:
(2) INFORMATION FOR SEQ ID NO:6:
   (i) SEQUENCE CHARACTERISTICS:
      (A) LENGTH: 434 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:6:
   Met Ser Glu Lys Thr Phe His Lys Ala Gln Thr Ile Arg Ala Lys Ala
    1               5                  10                  15
   Ser Gly Val Pro Ser Ile Val Glu Ala Val Gln Phe His Gly Val Arg
               20                  25                  30
   Ile Thr Lys Asn Asp Ala Leu Val Lys Glu Val Ser Glu Leu Tyr Arg
           35                  40                  45
   Ser Lys Asn Leu Asp Glu Leu Val His Asn Ser His Leu Ala Ala Arg
       50                  55                  60
   His Leu Gln Glu Val Gly Leu Met Asp Asn Ala Val Ala Leu Ile Asp
   65                  70                  75                  80
   Thr Ser Pro Ser Ser Asn Glu Gly Tyr Val Val Asn Phe Leu Val Arg
                   85                  90                  95
   Glu Pro Lys Ser Phe Thr Ala Gly Val Lys Ala Gly Val Ser Thr Asn
               100                 105                 110
   Gly Asp Ala Asp Val Ser Leu Asn Ala Gly Lys Gln Ser Val Gly Gly
           115                 120                 125
   Arg Gly Glu Ala Ile Asn Thr Gln Tyr Thr Tyr Thr Val Lys Gly Asp
       130                 135                 140
   His Cys Phe Asn Ile Ser Ala Ile Lys Pro Phe Leu Gly Trp Gln Lys
   145                 150                 155                 160
   Tyr Ser Asn Val Ser Ala Thr Leu Tyr Arg Ser Leu Ala His Met Pro
                   165                 170                 175
   Trp Asn Gln Ser Asp Val Asp Glu Asn Ala Ala Val Leu Ala Tyr Asn
               180                 185                 190
   Gly Gln Leu Trp Asn Gln Lys Leu Leu His Gln Val Lys Leu Asn Ala
           195                 200                 205
   Ile Trp Arg Thr Leu Arg Ala Thr Arg Asp Ala Ala Phe Ser Val Arg
       210                 215                 220
   Glu Gln Ala Gly His Thr Leu Lys Phe Ser Leu Glu Asn Ala Val Ala
   225                 230                 235                 240
   Val Asp Thr Arg Asp Arg Pro Ile Leu Ala Ser Arg Gly Ile Leu Ala
                   245                 250                 255
   Arg Phe Ala Gln Glu Tyr Ala Gly Val Phe Gly Asp Ala Ser Phe Val
               260                 265                 270
   Lys Asn Thr Leu Asp Leu Gln Ala Ala Ala Pro Leu Pro Leu Gly Phe
           275                 280                 285
   Ile Leu Ala Ala Ser Phe Gln Ala Lys His Leu Lys Gly Leu Gly Asp
       290                 295                 300
   Arg Glu Val His Ile Leu Asp Arg Cys Tyr Leu Gly Gly Gln Gln Asp
   305                 310                 315                 320
   Val Arg Gly Phe Gly Leu Asn Thr Ile Gly Val Lys Ala Asp Asn Ser
                   325                 330                 335
   Cys Leu Gly Gly Gly Ala Ser Leu Ala Gly Val Val His Leu Tyr Arg
               340                 345                 350
   Pro Leu Ile Pro Pro Asn Met Leu Phe Ala His Ala Phe Leu Ala Ser
           355                 360                 365
   Gly Ser Val Ala Ser Val His Ser Lys Asn Leu Val Gln Gln Leu Gln
       370                 375                 380
   Asp Thr Gln Arg Val Ser Ala Gly Phe Gly Leu Ala Phe Val Phe Lys
   385                 390                 395                 400
   Ser Ile Phe Arg Leu Glu Leu Asn Tyr Thr Tyr Pro Leu Lys Tyr Val
                   405                 410                 415
   Leu Gly Asp Ser Leu Leu Gly Gly Phe His Ile Gly Ala Gly Val Asn
               420                 425                 430
   Phe Leu
(2) INFORMATION FOR SEQ ID NO:7:
   (i) SEQUENCE CHARACTERISTICS:
      (A) LENGTH: 198 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:7:
   Met Leu Tyr Ile Leu Trp Lys Leu Asn Tyr Leu Gln Lys Lys Met Ser
    1              5                  10                  15
   Leu Arg Lys Ile Asn Phe Val Thr Gly Asn Val Lys Lys Leu Glu Glu
               20                  25                  30
   Val Lys Ala Ile Leu Lys Asn Phe Glu Val Ser Asn Val Asp Val Asp
           35                  40                  45
   Leu Asp Glu Phe Gln Gly Glu Pro Glu Phe Ile Ala Glu Arg Lys Cys
       50                  55                  60
   Arg Glu Ala Val Glu Ala Val Lys Gly Pro Val Leu Val Glu Asp Thr
   65                  70                  75                  80
   Ser Leu Cys Phe Asn Ala Met Gly Gly Leu Pro Gly Pro Tyr Ile Lys
                   85                  90                  95
   Trp Phe Leu Lys Asn Leu Lys Pro Glu Gly Leu His Asn Met Leu Ala
               100                 105                 110
   Gly Phe Ser Asp Lys Thr Ala Tyr Ala Gln Cys Ile Phe Ala Tyr Thr
           115                 120                 125
   Glu Gly Leu Gly Lys Pro Ile His Val Phe Ala Gly Lys Cys Pro Gly
       130                 135                 140
   Gln Ile Val Ala Pro Arg Gly Asp Thr Ala Phe Gly Trp Asp Pro Cys
   145                 150                 155                 160
   Phe Gln Pro Asp Gly Phe Lys Glu Thr Phe Gly Glu Met Asp Lys Asp
                   165                 170                 175
   Val Lys Asn Glu Ile Ser His Arg Ala Lys Ala Leu Glu Leu Leu Lys
               180                 185                 190
   Glu Tyr Phe Gln Asn Asn
           195
(2) INFORMATION FOR SEQ ID N0:8:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:8:
      CGAACACTTT ATATTTCTCG 20
(2) INFORMATION FOR SEQ ID NO:9:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:9:
      GATAGTTCCC TTCGTTCGGG 20
(2) INFORMATION FOR SEQ ID NO:10:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:10:
      TTTCTGGATT TTAACCTTCC 20
(2) INFORMATION FOR SEQ ID NO:11:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:11:
      TTTCCGAGAA GTCACGTTGG 20
(2) INFORMATION FOR SEQ ID NO:12:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:12:
      TACAGGAATT TTTGAACGGG 20
(2) INFORMATION FOR SEQ ID NO:13:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:13:
      CTTCAGATGA CGTGGATTCC 20
(2) INFORMATION FOR SEQ ID NO:14:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:14:
      GGAATCCGAA AAAGTGAACT 20
(2) INFORMATION FOR SEQ ID NO:15:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:15:
      AAGAGATACA CTCAATGGGG 20
(2) INFORMATION FOR SEQ ID NO:16:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID N0:16:
      ATCGATACCA CCGTCTCTGG 20
(2) INFORMATION FOR SEQ ID N0:17:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:17:
      TTGAATCTAC ACTAATCACC 20
(2) INFORMATION FOR SEQ ID NO:18:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:18:
      CCAATTATCT TTTCCAGTCA 20
(2) INFORMATION FOR SEQ ID NO:19:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:19:
      ACATTATAAA GTTACTGTCC 20
(2) INFORMATION FOR SEQ ID NO:20:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:20:
      TTTTAGTTAA AGCATTGACC 20
(2) INFORMATION FOR SEQ ID NO:21:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:21:
      ACATCTTTAT CCATTTCTCC 20
(2) INFORMATION FOR SEQ ID NO:22:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:22:
      TGCAAAGGCT CTGGAACTCC 20
(2) INFORMATION FOR SEQ ID NO:23:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:23:
      AAAAACCACT TGATATAAGG 20
(2) INFORMATION FOR SEQ ID NO:24:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:24:
      CATCCAAAAG CAGTATCACC 20
(2) INFORMATION FOR SEQ ID NO:25:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:25:
      TTAATTGGAT GCAAGCACCC C 21
(2) INFORMATION FOR SEQ ID NO:26:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:26:
      ATTACTATAC GAACATTTCC 20
(2) INFORMATION FOR SEQ ID NO:27:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:27:
      TTGTAAAGGC GTTAGTTTGG 20
(2) INFORMATION FOR SEQ ID NO:28:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:28:
      CAGGAGTATT TGGTGATGCG 20
(2) INFORMATION FOR SEQ ID NO:29:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:29:
      CGACGGGGAG AAGGTGACGG 20
(2) INFORMATION FOR SEQ ID NO:30:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:30:
      AAAACTTCTA CCAACAATGG 20
(2) INFORMATION FOR SEQ ID NO:31:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:31:
      CGTAATCTCT CTCGATTAGC 20
(2) INFORMATION FOR SEQ ID NO:32:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:32:
      CCGTGGGATG GCTACTTGCC 20
(2) INFORMATION FOR SEQ ID NO:33:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:33:
      TGGATTTGTG GCACGAGCGG 20
(2) INFORMATION FOR SEQ ID NO:34:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:34:
      TTGATTGCCT CTCCTCGTCC 20
(2) INFORMATION FOR SEQ ID NO:35:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:35:
      ATCAACATCT GATTGATTCC 20
(2) INFORMATION FOR SEQ ID NO:36:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:36:
      CAGCGAGCGC ATGCAACTAT ATATTGAGCA GG 32
(2) INFORMATION FOR SEQ ID NO:37:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (xi) SEQUENCE DESCRIPTION: SEQ ID NO:37:
      AATAAATATT TAAATATTCA GATATACCCT GAACTCTACA G 41
(2) INFORMATION FOR SEQ ID NO:38:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (xi) SEQUENCE DESCRIPTION: SEQ ID NO:38:
      AAACTGTAGA GTTCAGGGTA TATCTGAATA TTTAAATATT TATTC 45
(2) INFORMATION FOR SEQ ID NO:39:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:39:
      GTACGTGGAG CTCTGCAACT ATATATTGAG CAGG 34
(2) INFORMATION FOR SEQ ID NO:40:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:40:
      ATGACACTGC AGGATAGTTC CCTTCGTTCG GG 32
(2) INFORMATION FOR SEQ ID NO:41:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:41:
      GTGTTGCATC AGTTCATTCC 20
(2) INFORMATION FOR SEQ ID NO:42:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:42:
      GCTGTGCTAG AAGTCAGAGG 20
(2) INFORMATION FOR SEQ ID NO:43:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:43:
      GTTCTCCTTG GAATTCATCC 20
(2) INFORMATION FOR SEQ ID NO:44:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:44:
      AGTATATCTA GATGTGCGAG TCTCTGCCAA TT 32
(2) INFORMATION FOR SEQ ID NO:45:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:45:
      AGTAATTGTA CATTTAGTGG 20
(2) INFORMATION FOR SEQ ID NO:46:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:46:
      ATTAACCTTA CTTACTTACC 20
(2) INFORMATION FOR SEQ ID NO:47:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:47:
      CTAAACTAAG TAATATAACC 20
(2) INFORMATION FOR SEQ ID NO:48:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:48:
      GTTGATTCTT TGAGCACTGG 20
(2) INFORMATION FOR SEQ ID NO:49:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:49:
      AATTCGACCA ATTACATTGG 20
(2) INFORMATION FOR SEQ ID NO:50:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:50:
      AACATAGTTG TTGAGGAAGG 20
(2) INFORMATION FOR SEQ ID NO:51:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:51:
      AATTAATGGA GATTCTACGG 20
(2) INFORMATION FOR SEQ ID NO:52:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:52:
      TCAGCATCTA GAAATGCAGG 20
(2) INFORMATION FOR SEQ ID NO:53:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:53:
      CGAATGTCAA CATTCACTGG 20
(2) INFORMATION FOR SEQ ID NO:54:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:54:
      CTTAACCTGA TGTGTACTCG 20
(2) INFORMATION FOR SEQ ID NO:55:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:55:
      ATGAAGCTTT AGAGGATGCC 20
(2) INFORMATION FOR SEQ ID NO:56:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:56:
      CGACGAATTT CTGGAGTCGG 20
(2) INFORMATION FOR SEQ ID NO:57:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID N0:57:
      ACTGCATTAT CCATTAATCC 20
(2) INFORMATION FOR SEQ ID NO:58:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:58:
      CACCCAAATA ACATCTATCC 20
(2) INFORMATION FOR SEQ ID NO:59:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:59:
      TTTAACCTCA TCTTCGCTGG 20
(2) INFORMATION FOR SEQ ID NO:60:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:60:
      ATGTTCCGCA AGCTTGGTTC 20
(2) INFORMATION FOR SEQ ID NO:61:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:61:
      TTTAATTACC CAAGTTTGAG 20
(2) INFORMATION FOR SEQ ID NO:62:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:62: